# EUROPEAN PATENT SPECIFICATION

(11) **EP 2 981 246 B1**
(45) Date of publication and mention of the grant of the patent: **26.04.2023**
(21) Application number: 14730622.9
(22) Date of filing: 04.04.2014
(51) Int. Cl.: A61K 8/34, A61Q 5/02, A61K 8/60, A61Q 5/06, A61Q 5/12, A61K 8/25, A61K 8/37, A61K 8/85, A61K 8/92, A61K 8/06, A61K 8/81

(54) **HAIR CARE COMPOSITION COMPRISING A PRE-EMULSIFIED FORMULATION**
HAARPFLEGEMITTEL ENTHALTEND EINE PRE-EMULGIERTE FORMULIERUNG
COMPOSITION DE SOIN CAPILLAIRE COMPRENANT UNE FORMULATION PRE-EMULSIFIEE

(30) Priority: 05.04.2013 US 201361809023 P
(43) Date of publication of application: 10.02.2016
(73) Proprietor: The Procter & Gamble Company, Cincinnati, OH 45202 (US)
(72) Inventor: CARTER, John, David, Cincinnati, Ohio 45202 (US); STELLA, Qing, Cincinnati, Ohio 45202 (US); IWATA, Toshiyuki, Singapore 138547 (SG); CIENGSHIN, Tanashaya, Bangkok 10800 (TH); HOSSEINPOUR, Dariush, Cincinnati, Ohio 45202 (US); UEHARA, Nobuaki, Singapore 138547 (SG); OKADA, Toshiyuki, Singapore 138547 (SG); TAY, Raymond, Xu Zhan, Singapore 138547 (SG)
(74) Representative: P&G Patent Germany
(86) International application number: PCT/US2014/032907
(87) International publication number: WO 2014/165722

(56) References cited:
- WO-A1-99/24003
- WO-A2-2009/107062
- US-A1- 2002 168 327
- US-A1- 2007 110 696
- US-A1- 2007 128 147
- US-A1- 2009 220 443
- "DELIVERING SOLUTIONS, CREATING VALUE", INTERNET CITATION, 25 June 2009 (2009-06-25), pages 1-http://ww, XP002533982, Retrieved from the Internet: URL:www.pgchemicals.com [retrieved on 2009-06-25]

## Description

### FIELD OF THE INVENTION

The present invention relates to a hair care composition comprising a pre-emulsified emulsion selected from the group comprising methathesized unsaturated polyol esters, that are oligomers, wherein said one or more oligomers is a triglyceride oligomer, and wherein the triglyceride oligomer is a soy oligomer; wherein the composition further comprises an emulsifier, and wherein the composition is stable with respect to emulsion particle size, viscosity and visual phase separation and methods of using the same.

### BACKGROUND OF THE INVENTION

Human hair becomes soiled due to its contact with the surrounding environment and from the sebum secreted by the scalp. The soiling of hair causes it to have a dirty feel and an unattractive appearance.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils.

A variety of approaches have been developed to alleviate these after-shampoo problems. One approach is the application of a conditioner after shampooing.

In order to provide hair conditioning benefits after shampooing, a wide variety of conditioning actives have been proposed. These conditioning agents are known to enhance hair shine and provide moistness, softness, and static control to the hair. However, such components can also provide stickiness, greasy, or waxy feeling, particularly when the hair is dried.

Silicone conditioning agents are also known to provide conditioning benefits such as smoothness and combing ease due to the low surface tension of silicone compounds. However, silicone conditioning agents can cause dry feel or frizzy condition to the hair, again, particularly when the hair is dried. Additionally, the rising costs and the petroleum based nature of silicone have minimized silicone's desirability as a conditioning active. WO 2009/107062 A2 discloses hair care compositions for delivering a conditioning benefit.

Based on the foregoing, there is a need for a conditioning active which can provide conditioning benefits to hair and can replace, or be used in combination with silicone, or other conditioning actives, to maximize the conditioning activity of hair care compositions. Additionally, there is a desire to find a conditioning active which can be derived from a natural source, thereby providing a conditioning active derived from a renewable resource. There is also a desire to find a conditioning active that is both derived from a natural source and leads to a stable product. Numerous conditioning actives derived from a natural source have been used in personal care compositions. However, the compositions often show poor consumer benefits when insufficient amount is deposited especially from rinse off products like shampoo, conditioner and body wash. Furthermore, large particle sizes of the actives lead to uneven deposition that cause poor smoothness of skin and hair surfaces, poor combability and reduced volume for hair. There is a desire to enhance the deposition quality (film smoothness), skin/hair smoothness and hair volume of these conditioning actives to provide consumer noticeable benefits.

### SUMMARY OF THE INVENTION

The present invention is directed to the combination of the use of conditioning actives selected from the group comprising metathesized unsaturated polyol esters, sucrose polyesters, fatty esters with a molecular weight of 1500 or higher and their pre-emulsions with a particle size of from about 20 nanometers to 20 microns, in an embodiment from about 0.1-5µm significantly improves hair/skin conditioning benefits, active deposition and hair volume. The present invention is directed to a hair care composition comprising from about 0.25% to about 80% of a pre-emulsified emulsion comprising from about 0.005% to about 80% of metathesized unsaturated polyol esters that are oligomers, wherein said one or more oligomers is a triglyceride oligomer, and wherein the triglyceride oligomer is a soy oligomer, having molecular weight of 1500 or higher by weight of said hair care composition; wherein an emulsifier is selected from the group consisting of anionic, non-ionic, cationic, amphoteric and mixtures thereof wherein the average particle size of the pre-emulsified oil in water emulsion is from about 20 nanometer to 20 microns; a cationic surfactant system and wherein the composition is stable with respect to emulsion particle size, viscosity and visual phase separation.

These and other features, aspects, and advantages of the present invention will become evident to those skilled in the art from a reading of the present disclosure.

### DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at 25 °C and at ambient conditions, where "ambient conditions" means conditions under about one atmosphere of pressure and at about 50% relative humidity. All such weights as they pertain to listed ingredients are based on the active level and do not include carriers or by-products that may be included in commercially available materials, unless otherwise specified.

The term "comprising," as used herein, means that other steps and other ingredients which do not affect the end result can be added. This term encompasses the terms "consisting of" and "consisting essentially of." The compositions and methods/processes of the present invention can comprise, consist of, and consist essentially of the elements and limitations of the invention described herein, as well as any of the additional or optional ingredients, components, steps, or limitations described herein.

The terms "include," "includes," and "including," as used herein, are meant to be non-limiting and are understood to mean "comprise," "comprises," and "comprising," respectively.

The test methods disclosed in the Test Methods Section of the present application should be used to determine the respective values of the parameters of Applicants' inventions.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated. The term "weight percent" may be denoted as "wt.%" herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### A. Emulsion

The term "pre-emulsion" in this patent application describes any stable emulsion or dispersion of a conditioning material such as oil, viscous liquid, viscoelastic liquid, or solid in an aqueous medium, separately prepared and used as one of the components of a personal care composition.

The same pre-emulsion can be used as a component of different personal care products provided that it is compatible with the other components of the personal care products.

Stable means that the viscosity, particle size, and other important characteristics of the emulsion do not significantly change over reasonable time under exposure to typical temperature, moisture, pressure, shear, light and other environmental conditions that the pre-emulsion is exposed during packing, storage, and transportation.

Historically, naturals and natural derivatives are used primarily as image ingredients in personal care applications due to their instability in chasses especially high surfactant systems. The use of active emulsions presents 3 advantages in the present invention: i) Additional emulsifiers in the emulsion reduce the interactions of actives with the surfactants in the chassis, which in turn enhances product stability; ii) Emulsified actives, especially those with higher viscosities, potentially improve spreadibility on hair surfaces with different properties (e.g. virgin vs. damaged hair); iii) Emulsions significantly affect the appearance of a clear chassis. Emulsions with particle sizes in the range of 100-500 nm alters clear to translucent appearance, which consumers perceive as more benefit ingredients in the product.

### a. Metathesized Oligomer

The hair care composition may comprise from about 0.02% to about 15%, alternatively from about 0.1% to about 10%, and alternatively from about 0.25% to about 5%, of one or more oligomers derived from metathesis of unsaturated polyol esters, by weight of said hair care composition. Exemplary metathesized unsaturated polyol esters and their starting materials are set forth in U.S. Patent Application U.S. 2009/0220443 A1.

A metathesized unsaturated polyol ester refers to the product obtained when one or more unsaturated polyol ester ingredient(s) are subjected to a metathesis reaction. Metathesis is a catalytic reaction that involves the interchange of alkylidene units among compounds containing one or more double bonds (i.e., olefinic compounds) via the formation and cleavage of the carbon-carbon double bonds. Metathesis may occur between two of the same molecules (often referred to as self-metathesis) and/or it may occur between two different molecules (often referred to as cross-metathesis). Self-metathesis may be represented schematically as shown in Equation I:

R¹-CH=CH-R²+R¹-CH=CH-R²↔ R¹-CH=CH-R¹+R²-CH=CH-R² (I)

where R¹ and R² are organic groups.

Cross-metathesis may be represented schematically as shown in Equation II:

R¹-CH=CH=R²+R³-CH=CH-R⁴ R¹-CH=CH-R³+R-CH=CH-R⁴+R²-CH=CH-R³+R²-CH=CH-R⁴+R¹-CH=CH-R¹+R²-CH=CH-R²+R³-CH=CH-R³+R⁴-CH=CH-R⁴ (II)

where R¹, R², R³, and R⁴ are organic groups.

When the unsaturated poyol ester comprises molecules that have more than one carbon-carbon double bond (i.e., a polyunsaturated polyol ester), self-metathesis results in oligomerization of the unsaturated polyol ester. The self-metathesis reaction results in the formation of metathesis dimers, metathesis trimers, and metathesis tetramers. Higher order metathesis oligomers, such as metathesis pentamers and metathesis hexamers, may also be formed by continued self-metathesis and will depend on the number and type of chains connecting the unsaturated polyol ester material as well as the number of esters and orientation of the ester relative to the unsaturation

As a starting material, metathesized unsaturated polyol esters are prepared from one or more unsaturated polyol esters. As used herein, the term "unsaturated polyol ester" refers to a compound having two or more hydroxyl groups wherein at least one of the hydroxyl groups is in the form of an ester and wherein the ester has an organic group including at least one carbon-carbon double bond. In many embodiments, the unsaturated polyol ester can be represented by the general structure I: 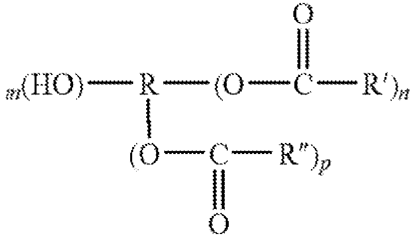 where n≥1; m ≥0; p≥0; (n+m+p)≥2; R is an organic group; R' is an organic group having at least one carbon-carbon double bond; and R" is a saturated organic group. Exemplary embodiments of the unsaturated polyol ester are described in detail in U.S. 2009/0220443 A1.

Also disclosed herein are unsaturated esters of glycerol. Sources of unsaturated polyol esters of glycerol include synthesized oils, natural oils (e.g., vegetable oils, algae oils, bacterial derived oils, and animal fats), combinations of these, and the like. Recycled used vegetable oils may also be used. Representative examples of vegetable oils include argan oil, canola oil, rapeseed oil, coconut oil, corn oil, cottonseed oil, olive oil, palm oil, peanut oil, safflower oil, sesame oil, soy-bean oil, sunflower oil, high oleoyl soy-bean oil, high oleoyl sunflower oil, linseed oil, palm kernel oil, tung oil, castor oil, high erucic rape oils, Jatropha oil, combinations of these, and the like. Representative examples of animal fats include lard, tallow, chicken fat, yellow grease, fish oil, combinations of these, and the like. A representative example of synthesized oil includes tall oil, which is a byproduct of wood pulp manufacture.

Other examples of unsaturated polyol esters include diesters such as those derived from ethylene glycol or propylene glycol, esters such as those derived from pentaerythritol or dipentaerythritol, or sugar esters such as SEFOSE^{®}. Sugar esters such as SEFOSE^{®} include one or more types of sucrose polyesters, with up to eight ester groups that could undergo a metathesis exchange reaction. Sucrose polyesters are derived from a natural resource and therefore, the use of sucrose polyesters can result in a positive environmental impact. Sucrose polyesters are polyester materials, having multiple substitution positions around the sucrose backbone coupled with the chain length, saturation, and derivation variables of the fatty chains. Such sucrose polyesters can have an esterification ("IBAR") of greater than about 5. The sucrose polyester may have an IBAR of from about 5 to about 8, or the sucrose polyester has an IBAR of about 5-7, or the sucrose polyester has an IBAR of about 6, or the sucrose polyester has an IBAR of about 8. As sucrose polyesters are derived from a natural resource, a distribution in the IBAR and chain length may exist. For example a sucrose polyester having an IBAR of 6, may contain a mixture of mostly IBAR of about 6, with some IBAR of about 5 and some IBAR of about 7. Additionally, such sucrose polyesters may have a saturation or iodine value ("IV") of about 3 to about 140, or an IV of about 10 to about 120, or an IV of about 20 to 100. Further, such sucrose polyesters have a chain length of about C₁₂ to C₂₀ but are not limited to these chain lengths.

Non-limiting examples of sucrose polyesters suitable for use include SEFOSE^{®} 1618S, SEFOSE^{®} 1618U, SEFOSE^{®} 1618H, Sefa Soyate IMF 40, Sefa Soyate LP426, SEFOSE^{®} 2275, SEFOSE^{®} C1695, SEFOSE^{®} C18:0 95, SEFOSE^{®} C1495, SEFOSE^{®} 1618H B6, SEFOSE^{®} 1618S B6, SEFOSE^{®} 1618U B6, Sefa Cottonate, SEFOSE^{®} C1295, Sefa C895, Sefa C1095, SEFOSE^{®} 1618S B4.5, all available from The Procter and Gamble Co. of Cincinnati, Ohio.

Other examples of natural polyol esters may include but not be limited to sorbitol esters, maltitol esters, sorbitan esters, maltodextrin derived esters, xylitol esters, and other sugar derived esters.

Chain lengths of esters are not restricted to C8-C22 or even chain lengths only and can include natural esters that come from co-metathesis of fats and oils with short chain olefins both natural and synthetic providing a polyol ester feedstock which can have even and odd chains as well as shorter and longer chains for the selfmetathesis reaction. Suitable short chain olefins include ethylene and butene.

The oligomers derived from the metathesis of unsaturated polyol esters may be further modified via hydrogenation. For example, in certain embodiments, the oligomer can be about 60% hydrogenated or more; in certain embodiments, about 70% hydrogenated or more; in certain embodiments, about 80% hydrogenated or more; in certain embodiments, about 85% hydrogenated or more; in certain embodiments, about 90% hydrogenated or more; and in certain embodiments, generally 100% hydrogenated.

The triglyceride oligomer is derived from the self-metathesis of soybean oil. The soy oligomer can include hydrogenated soy polyglycerides. The soy oligomer may also include C₁₅-C₂₃ alkanes, as a byproduct. An example of metathesisderived soy oligomers is the fully hydrogenated DOW CORNING^{®} HY-3050 soy wax, available from Dow Corning.

In other embodiments, the metathesized unsaturated polyol esters can be used as a blend with one or more non-metathesized unsaturated polyol esters. The non-metathesized unsaturated polyol esters can be fully or partially hydrogenated. Such an example is DOW CORNING^{®} HY-3051, a blend of HY-3050 oligomer and hydrogenated soybean oil (HSBO), available from Dow Corning. In some embodiments of the invention, the non-metathesized unsaturated polyol ester is an unsaturated ester of glycerol. Sources of unsaturated polyol esters of glycerol include synthesized oils, natural oils (e.g., vegetable oils, algae oils, bacterial-derived oils, and animal fats), combinations of theses, and the like. Recycled used vegetable oils may also be used. Representative examples of vegetable oils include those listed above.

Other modifications of the polyol ester oligomers can be partial amidation of some fraction of the esters with ammonia or higher organic amines such as dodecyl amine or other fatty amines. This modification will alter the overall oligomer composition but can be useful in some applications providing increased lubricity of the product. Another modification can be via partial amidation of a poly amine providing potential for some pseudo cationic nature to the polyol ester oligomers. Such an example is DOW CORNING^{®} material HY-3200. Other exemplary embodiments of amido functionalized oligomers are described in detail in WO2012006324A1.

The poloyl ester oligomers may be modified further by partial hydroformylation of the unsaturated functionality to provide one or more OH groups and an increase in the oligomer hydrophilicity.

In other embodiments, the unsaturated polyol esters and blends can be modified prior to oligomerization to incorporate near terminal branching. Exemplary polyol esters modified prior to oligomerization to incorporate terminal branching are set forth in WO2012/009525A2.

### a. Non-metathesized Sugar polyesters

The personal care composition may also comprise from about 0.05% to about 15%, alternatively from about 0.1% to about 10%, and alternatively from about 0.25% to about 5%, of one or more of sugar polyesters, by weight of said personal care composition. Typical examples of sucrose polyesters such as SEFOSE^{®}. The sucrose molecule can be esterified in one or more of its eight hydroxyl groups with saturated or unsaturated carboxylic acids, providing a very diverse set of possible molecular structures of polyesters. The non-metathesized unsaturated sucrose polyesters or saturated sucrose polyesters and their mixtures can also be used as conditioning material in hair care and body wash compositions.

### b. Mixtures of Conditioning materials

The personal care composition may also comprise of one or more materials selected from the group of metathesized oligomers, sucrose polyesters, other fatty esters, or other conditioning materials (silicone or non-silicone).

### B. Emulsifiers

Emulsifiers selection for each conditioning active is guided by the Hydrophilic-Lipophilic-Balance value (HLB value) of emulsifiers. Suitable range of HLB value is 6-16, more preferably 8-14. Emulsifiers with an HLB higher than 10 are water soluble. Emulsifiers with low HLB are lipid soluble. To obtain suitable HLB value, a mixture of two or more emulsifiers may be used. Suitable emulsifiers include non-ionic, cationic, anionic and amphoteric emulsifiers.

The concentration of the emulsifier in the emulsion should be sufficient to provide the desired emulsification of the conditioning actives to achieve desired particle sizes and emulsion stability, and generally ranges from about 0.1 wt.%-about 50 wt%, from about 1 wt.%-about 30 wt.%, from about 2 wt.%-about 20 wt.%, for example.

Non-ionic emulsifiers suitable for use in the emulsion may include a wide variety of emulsifiers useful herein and include, but not limited to, those selected from the group consisting of sorbitan esters, glyceryl esters, polyglyceryl esters, methyl glucose esters, sucrose esters, ethoxylated fatty alcohols, hydrogenated castor oil ethoxylates, sorbitan ester ethoxylates, polymeric emulsifiers, and silicone emulsifiers.

Sorbitan esters are useful in the present invention. Preferable are sorbitan esters of C16-C22 saturated, unsaturated and branched-chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN(Registered trademark) 80), sorbitan sesquioleate (e.g., Arlacel(Registered trademark) 83), sorbitan monoisostearate (e.g., CRILL(Registered trademark) 6 made by Croda), sorbitan stearates (e.g., SPAN(Registered trademark) 60), sorbitan triooleate (e.g., SPAN(Registered trademark) 85), sorbitan tristearate (e.g., SPAN(Registered trademark) 65), sorbitan dipalmitates (e.g., SPAN(Registered trademark) 40), and sorbitan isostearate. Sorbitan monoisostearate and sorbitan sesquioleate are particularly preferred emulsifiers for use in the present invention.

Other suitable emulsifiers for use in the present invention include, but is not limited to, glyceryl monoesters, preferably glyceryl monoesters of C16-C22 saturated, unsaturated and branched chain fatty acids such as glyceryl oleate, glyceryl monostearate, glyceryl monopalmitate, glyceryl monobehenate, and mixtures thereof; polyglyceryl esters of C16-C22 saturated, unsaturated and branched-chain fatty acids, such as polyglyceryl-4 isostearate, polyglyceryl-3 oleate, diglycerol monooleate, tetraglycerol monooleate and mixtures thereof; methyl glucose esters, preferably methyl glucose esters of C16-C22 saturated, unsaturated and branched-chain fatty acids such as methyl glucose dioleate, methyl glucose sesquiisostearate, and mixtures thereof; sucrose fatty acid esters, preferably sucrose esters of C12-C22 saturated, unsaturated and branched-chain fatty acids such as sucrose stearate, sucrose trilaurate, sucrose distearate (e.g., Crodesta(Registered trademark) F10), and mixtures thereof; C12-C22 ethoxylated fatty alcohols such as oleth-2, oleth-3, steareth-2, and mixtures thereof; hydrogenated castor oil ethoxylates such as PEG-7 hydrogenated castor oil; sorbitan ester ethoxylates such as PEG-40 sorbitan peroleate, Polysorbate-80, and mixtures thereof; polymeric emulsifiers such as ethoxylated dodecyl glycol copolymer; and silicone emulsifiers such as laurylmethicone copolyol, cetyldimethicone, dimethicone copolyol, and mixtures thereof.

Anionic emulsifiers are suitable for use in the emulsion of the present invention. A variety of anionic emulsifiers can be used in the hair care composition as described herein. The anionic emulsifiers include, by way of illustrating and not limitation, water-soluble salts of alkyl sulfates, alkyl ether sulfates, alkyl isothionates, alkyl carboxylates, alkyl sulfosuccinates, alkyl succinamates, alkyl sulfate salts such as sodium dodecyl sulfate, alkyl sarcosinates, alkyl derivatives of protein hydrolyzates, acyl aspartates, alkyl or alkyl ether or alkylaryl ether phosphate esters, sodium dodecyl sulphate, phospholipids or lecithin, or soaps, sodium, potassium or ammonium stearate, oleate or palmitate, alkylarylsulfonic acid salts such as sodium dodecylbenzenesulfonate, sodium dialkylsulfosuccinates, dioctyl sulfosuccinate, sodium dilaurylsulfosuccinate, poly(styrene sulfonate) sodium salt, isobutylene-maleic anhydride copolymer, gum arabic, sodium alginate, carboxymethylcellulose, cellulose sulfate and pectin, poly(styrene sulfonate), isobutylene-maleic anhydride copolymer, gum arabic, carrageenan, sodium alginate, pectic acid, tragacanth gum, almond gum and agar; semi-synthetic polymers such as carboxymethyl cellulose, sulfated cellulose, sulfated methylcellulose, carboxymethyl starch, phosphated starch, lignin sulfonic acid; and synthetic polymers such as maleic anhydride copolymers (including hydrolyzates thereof), polyacrylic acid, polymethacrylic acid, acrylic acid butyl acrylate copolymer or crotonic acid homopolymers and copolymers, vinylbenzenesulfonic acid or 2-acrylamido-2-methylpropanesulfonic acid homopolymers and copolymers, and partial amide or partial ester of such polymers and copolymers, carboxymodified polyvinyl alcohol, sulfonic acid-modified polyvinyl alcohol and phosphoric acid-modified polyvinyl alcohol, phosphated or sulfated tristyrylphenol ethoxylates.

In addition, anionic emulsifiers that have acrylate functionality useful herein include, but aren't limited to: poly(meth)acrylic acid; copolymers of (meth)acrylic acids and its (meth)acrylates with C1-22 alkyl, C1-C8 alkyl, butyl; copolymers of (meth)acrylic acids and (meth)acrylamide; Carboxyvinylpolymer; acrylate copolymers such as Acrylate/C10-30 alkyl acrylate crosspolymer, Acrylic acid/vinyl ester copolymer/Acrylates/Vinyl Isodecanoate crosspolymer, Acrylates/Palmeth-25 Acrylate copolymer, Acrylate/Steareth-20 Itaconate copolymer, and Acrylate/Celeth-20 Itaconate copolymer; Polystyrene sulphonate, copolymers of methacrylic acid and acrylamidomethylpropane sulfonic acid, and copolymers of acrylic acid and acrylamidomethylpropane sulfonic acid; carboxymethycellulose; carboxy guar; copolymers of ethylene and maleic acid; and acrylate silicone polymer. Neutralizing agents may be included to neutralize the anionic emulsifiers herein. Non-limiting examples of such neutralizing agents include sodium hydroxide, potassium hydroxide, ammonium hydroxide, monoethanolamine, diethanolamine, triethanolamine, diisopropanolamine, aminomethylpropanol, tromethamine, tetrahydroxypropyl ethylenediamine, and mixtures thereof. Commercially available anionic emulsifiers include, for example, Carbomer supplied from Noveon under the tradename Carbopol 981 and Carbopol 980; Acrylates/C10-30 Alkyl Acrylate Crosspolymer having tradenames Pemulen TR-1, Pemulen TR-2, Carbopol 1342, Carbopol 1382, and Carbopol ETD 2020, all available from Noveon; sodium carboxymethylcellulose supplied from Hercules as CMC series; and Acrylate copolymer having a tradename Capigel supplied from Seppic. In another embodiment, anionic emulsifiers are carboxymethylcelluloses.

Cationic Emulsifers suitable for use in the emulsion of the present invention may include a wide variety of emulsifiers useful herein and include, but not limited to,: mono-long alkyl quaternized ammonium salt; a combination of mono-long alkyl quaternized ammonium salt and di-long alkyl quaternized ammonium salt; mono-long alkyl amidoamine salt; a combination of mono-long alkyl amidoamine salt and di-long alkyl quaternized ammonium salt, a combination of mono-long alkyl amindoamine salt and mono-long alkyl quaternized ammonium salt

The cationic emulsifier is included in the composition at a level by weight of from about 0.1% to about 10%, preferably from about 0.5% to about 8%, more preferably from about 0.8 % to about 5%, still more preferably from about 1.0% to about 4%.

### Mono-long alkyl quaternized ammonium salt

The monoalkyl quaternized ammonium salt cationic surfactants useful herein are those having one long alkyl chain which has from 12 to 30 carbon atoms, preferably from 16 to 24 carbon atoms, more preferably C18-22 alkyl group. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Mono-long alkyl quaternized ammonium salts useful herein are those having the formula (I): 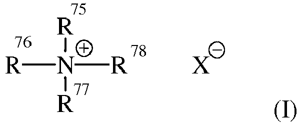 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Nonlimiting examples of such mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt.

### Mono-long alkyl amidoamine salt

Mono-long alkyl amines are also suitable as cationic surfactants. Primary, secondary, and tertiary fatty amines are useful. Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from about 1 : 0.3 to about 1 : 2, more preferably from about 1 : 0.4 to about 1:1.

### Di-long alkyl quaternized ammonium salt

Di-long alkyl quaternized ammonium salt is preferably combined with a mono-long alkyl quaternized ammonium salt or mono-long alkyl amidoamine salt. It is believed that such combination can provide easy-to rinse feel, compared to single use of a monoalkyl quaternized ammonium salt or mono-long alkyl amidoamine salt. In such combination with a mono-long alkyl quaternized ammonium salt or mono-long alkyl amidoamine salt, the di-long alkyl quaternized ammonium salts are used at a level such that the wt% of the dialkyl quaternized ammonium salt in the cationic surfactant system is in the range of preferably from about 10% to about 50%, more preferably from about 30% to about 45%.

The dialkyl quaternized ammonium salt cationic surfactants useful herein are those having two long alkyl chains having 12-30 carbon atoms, preferably 16-24 carbon atoms, more preferably 18-22 carbon atoms. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Di-long alkyl quaternized ammonium salts useful herein are those having the formula (II): 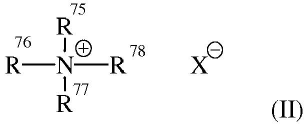 wherein two of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof. Such dialkyl quaternized ammonium salt cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride. Such dialkyl quaternized ammonium salt cationic surfactants also include, for example, asymmetric dialkyl quaternized ammonium salt cationic surfactants

Amphoteric emulsifiers suitable for use in the emulsion may include a wide variety of emulsifiers useful herein and include, but not limited to those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Exemplary amphoteric detersive surfactants for use in the present hair care composition include cocoamphoacetate, cocoamphodiacetate, lauroamphoacetate, lauroamphodiacetate, and mixtures thereof.

In addition to these primary emulsifiers, the compositions of the present invention can optionally contain a coemulsifier to provide additional water-lipid emulsion stability. Suitable coemulsifiers include, but are not limited to, phosphatidyl cholines and phosphatidyl choline-containing compositions such as lecithins; long chain C16-C22 fatty acid salts such as sodium stearate; long chain C16-C22 dialiphatic, short chain C1-C4 dialiphatic quaternary ammonium salts such as ditallow dimethyl ammonium chloride and ditallow dimethyl ammonium methylsulfate; long chain C16-C22 dialkoyl(alkenoyl)-2-hydroxyethyl, short chain C1-C4 dialiphatic quaternary ammonium salts such as ditallowoyl-2-hydroxyethyl dimethyl ammonium chloride; the long chain C16-C22 dialiphatic imidazolinium quaternary ammonium salts such as methyl-1-tallow amido ethyl-2-tallow imidazolinium methylsulfate and methyl-1-oleyl amido ethyl-2-oleyl imidazolinium methylsulfate; short chain C1-C4 dialiphatic, long chain C16-C22 monoaliphatic benzyl quaternary ammonium salts such as dimethyl stearyl benzyl ammonium chloride, and synthetic phospholipids such as stearamidopropyl PG-dimonium chloride (Phospholipid PTS from Mona Industries).

### C. EMULSION

In an embodiment of the present invention, it has found that the actives selected from the group comprising metathesized unsaturated polyol esters, sucrose polyesters, fatty esters with a molecular weight equal to and higher than 1500 provide higher conditioning benefit when they are pre-emulsified with a particle size in the range of about 20 nanometers to 20 microns, and in an embodiment from about 0.1-5µm compared to direct blend in. The use of active pre-emulsified emulsions presents 3 advantages: i) Additional emulsifiers in the emulsion reduce the interactions of active with the cationic surfactant structures; ii) The small particle size of the actives in emulsion leads to more even deposition and reduces island-like spotty deposits; and iii) The homogeneous deposition is more favorable to provide smoothness for hair/skin surfaces and easy combing and enhanced volume for hair.

### Method of making pre-emulsion

Making the emulsion comprising components below is to pre-emulsify the conditioning active before their addition to the hair care composition. A non-limiting example of a method of making is provided below. All oil soluble components are mixed in a vessel. Heat may be applied to allow mixture to liquidity. All water soluble components are mixed in a separate vessel and heated to about same temperature as the oil phase. The oil phase and aqueous phase are mixed under a high shear mixer (example, Turrax mixer by IKA). The particle size of the conditioning active is in the range of 0.01-5 µm, more preferred 0.05-1µm, most preferred 0.1-0.5 µm. High energy mixing device may be used to achieve desired particle size. High-energy mixing device include, but not limited to Microfluidizer from Microfluidics Corp., Sonolator from Sonic Corp., Colloid mill from Sonic Corp.

The stability of a composition can be measured by composition viscosity/rheology, particle size and visual observations of phase separation over a period of time via conventional methodologies. The length of time can be measured by days, weeks and months. Typical measuring temperatures are room temperature, e.g. about 25°C, and/or at elevated temperature, e.g. 40°C.

**In an embodiment of the present invention,** a hair care composition may comprise from about 0.25% to about 80% of a pre-emulsified emulsion, in a further embodiment from about 0.5% to about 80% of a pre-emulsified emulsion.

### D. CATIONIC SURFACTANT SYSTEM

The composition of the present invention comprises a cationic surfactant system. The cationic surfactant system can be one cationic surfactant or a mixture of two or more cationic surfactants. Preferably, the cationic surfactant system is selected from: mono-long alkyl quaternized ammonium salt; a combination of mono-long alkyl quaternized ammonium salt and di-long alkyl quaternized ammonium salt; mono-long alkyl amidoamine salt; a combination of mono-long alkyl amidoamine salt and di-long alkyl quaternized ammonium salt, a combination of mono-long alkyl amindoamine salt and mono-long alkyl quaternized ammonium salt

The cationic surfactant system is included in the composition at a level by weight of from about 0.1% to about 10%, preferably from about 0.5% to about 8%, more preferably from about 0.8 % to about 5%, still more preferably from about 1.0% to about 4%.

### Mono-long alkyl quaternized ammonium salt

The monoalkyl quaternized ammonium salt cationic surfactants useful herein are those having one long alkyl chain which has from 12 to 30 carbon atoms, preferably from 16 to 24 carbon atoms, more preferably C18-22 alkyl group. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Mono-long alkyl quaternized ammonium salts useful herein are those having the formula (I): 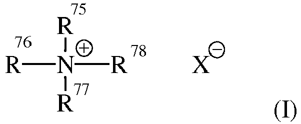 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Non-limiting examples of such mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt.

### Mono-long alkyl amidoamine salt

Mono-long alkyl amines are also suitable as cationic surfactants. Primary, secondary, and tertiary fatty amines are useful. Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from about 1 : 0.3 to about 1 : 2, more preferably from about 1 : 0.4 to about 1:1.

### Di-long alkyl quaternized ammonium salt

Di-long alkyl quaternized ammonium salt is preferably combined with a mono-long alkyl quaternized ammonium salt or mono-long alkyl amidoamine salt. It is believed that such combination can provide easy-to-rinse feel, compared to single use of a monoalkyl quaternized ammonium salt or mono-long alkyl amidoamine salt. In such combination with a mono-long alkyl quaternized ammonium salt or mono-long alkyl amidoamine salt, the di-long alkyl quaternized ammonium salts are used at a level such that the wt% of the dialkyl quaternized ammonium salt in the cationic surfactant system is in the range of preferably from about 10% to about 50%, more preferably from about 30% to about 45%.

The dialkyl quaternized ammonium salt cationic surfactants useful herein are those having two long alkyl chains having 12-30 carbon atoms, preferably 16-24 carbon atoms, more preferably 18-22 carbon atoms. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Di-long alkyl quaternized ammonium salts useful herein are those having the formula (II): 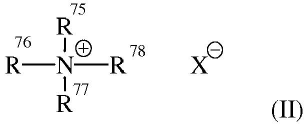 wherein two of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof. Such dialkyl quaternized ammonium salt cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride. Such dialkyl quaternized ammonium salt cationic surfactants also include, for example, asymmetric dialkyl quaternized ammonium salt cationic surfactants.

### C. HIGH MELTING POINT FATTY COMPOUND

The high melting point fatty compound useful herein has a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Non-limiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched-chain alcohols. Preferred fatty alcohols include, for example, cetyl alcohol, stearyl alcohol, behenyl alcohol, and mixtures thereof.

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

The high melting point fatty compound is included in the composition at a level of from about 0.1% to about 20%, preferably from about 1% to about 15%, more preferably from about 1.5% to about 8% by weight of the composition, in view of providing improved conditioning benefits such as slippery feel during the application to wet hair, softness and moisturized feel on dry hair.

### D. AQUEOUS CARRIER

The gel matrix of the hair care composition of the present invention includes an aqueous carrier. Accordingly, the formulations of the present invention can be in the form of pourable liquids (under ambient conditions). Such compositions will therefore typically comprise an aqueous carrier, which is present at a level of from about 20 wt.% to about 95 wt.%, or even from about 60 wt.% to about 85 wt.%. The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carrier useful in the present invention includes water and water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol. According to embodiments of the present invention, the hair care compositions may have a pH in the range from about 2 to about 10, at 25°C. In one embodiment, the hair care composition has a pH in the range from about 2 to about 6, which may help to solubilize minerals and redox metals already deposited on the hair. Thus, the hair care composition can also be effective toward washing out the existing minerals and redox metals deposits, which can reduce cuticle distortion and thereby reduce cuticle chipping and damage.

### E. GEL MATRIX

The composition of the present invention comprises a gel matrix. The gel matrix comprises a cationic surfactant, a high melting point fatty compound, and an aqueous carrier.

The gel matrix is suitable for providing various conditioning benefits such as slippery feel during the application to wet hair and softness and moisturized feel on dry hair. In view of providing the above gel matrix, the cationic surfactant and the high melting point fatty compound are contained at a level such that the weight ratio of the cationic surfactant to the high melting point fatty compound is in the range of, preferably from about 1:1 to about 1:10, more preferably from about 1:1 to about 1:6.

### C. Additional Components

### 1. Silicone Conditioning Agent

According to embodiments of the present invention, the hair care composition includes a silicone conditioning agent which comprises a silicone compound. The silicone compound may comprise volatile silicone, non-volatile silicones, or combinations thereof. In one aspect, non-volatile silicones are employed. If volatile silicones are present, it will typically be incidental to their use as a solvent or carrier for commercially available forms of non-volatile silicone materials ingredients, such as silicone gums and resins. The silicone compounds may comprise a silicone fluid conditioning agent and may also comprise other ingredients, such as a silicone resin to improve silicone fluid deposition efficiency or enhance glossiness of the hair. The concentration of the silicone compound in the conditioner composition typically ranges from about 0.01 wt.% to about 10 wt.%, from about 0.1 wt.% to about 8 wt.%, from about 0.1 wt.% to about 5 wt.%, or even from about 0.2 wt.% to about 3 wt.%, for example

Exemplary silicone compounds include (a) a first polysiloxane which is non-volatile, substantially free of amino groups, and has a viscosity of from about 100,000 mm²s⁻¹ to about 30,000,000 mm²s⁻¹; (b) a second polysiloxane which is non-volatile, substantially free of amino groups, and has a viscosity of from about 5 mm²s⁻¹ to about 10,000 mm²s⁻¹; (c) an aminosilicone having less than about 0.5 wt% nitrogen by weight of the aminosilicone; (d) a silicone copolymer emulsion with an internal phase viscosity of greater than about 100×10⁶ mm²s⁻¹, as measured at 25°C; (e) a silicone polymer containing quaternary groups; or (f) a grafted silicone polyol, wherein the silicone compounds (a) - (f) are disclosed in U.S. Patent Application Publication Nos. 2008/0292574, 2007/0041929, 2008/0292575, and 2007/0286837, each of which is incorporated by reference herein in its entirety.

### a. First Polysiloxane

The hair care composition of the present invention may comprise a first polysiloxane. The first polysiloxane is non-volatile, and substantially free of amino groups. In the present invention, the first polysiloxanes being "substantially free of amino groups" means that the first polysiloxane contains 0 wt.% of amino groups. The first polysiloxane has a viscosity of from about 100,000 mm²s⁻¹ to about 30,000,000 mm²s⁻¹ at 25°C. For example, the viscosity may range from about 300,000 mm²s⁻¹ to about 25,000,000 mm²s⁻¹, or from about 10,000,000 mm²s⁻¹ to about 20,000,000 mm²s⁻¹. The first polysiloxane has a molecular weight from about 100,000 to about 1,000,000. For example, the molecular weight may range from about 130,000 to about 800,000, or from about 230,000 to about 600,000. According to one aspect, the first polysiloxane may be nonionic.

Exemplary first non-volatile polysiloxanes useful herein include those in accordance with the following the general formula (I): 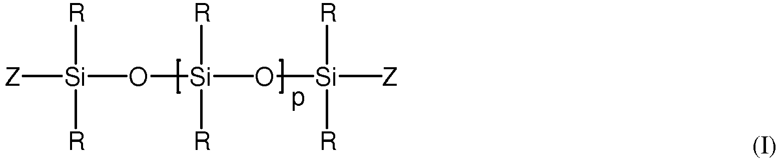 wherein R is alkyl or aryl, and p is an integer from about 1,300 to about 15,000, such as from about 1,700 to about 11,000, or from about 3,000 to about 8,000. Z represents groups which block the ends of the silicone chains. The alkyl or aryl groups substituted on the siloxane chain (R) or at the ends of the siloxane chains Z can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. According to an embodiment, suitable Z groups include hydroxy, methyl, methoxy, ethoxy, propoxy, and aryloxy. The two R groups on each silicon atom may represent the same group or different groups. According to one embodiment, the two R groups may represent the same group. Suitable R groups include methyl, ethyl, propyl, phenyl, methylphenyl and phenylmethyl. Exemplary silicone compounds include polydimethylsiloxane, polydiethylsiloxane, and polymethylphenylsiloxane. According to one embodiment, polydimethylsiloxane is the first polysiloxane. Commercially available silicone compounds useful herein include, for example, those available from the General Electric Company in their TSF451 series, and those available from Dow Coming in their Dow Corning SH200 series.

The silicone compounds that can be used herein also include a silicone gum. The term "silicone gum", as used herein, means a polyorganosiloxane material having a viscosity at 25°C of greater than or equal to 1,000,000 mm²s⁻¹. It is recognized that the silicone gums described herein can also have some overlap with the above-disclosed silicone compounds. This overlap is not intended as a limitation on any of these materials. The "silicone gums" will typically have a mass molecular weight in excess of about 165,000, generally between about 165,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. Commercially available silicone gums useful herein include, for example, TSE200A available from the General Electric Company.

### b. Second Polysiloxane

The hair care composition of the present invention may comprise a second polysiloxane. The second polysiloxane is non-volatile, and substantially free of amino groups. In the present invention, the second polysiloxane being "substantially free of amino groups" means that the second polysiloxane contains 0 wt.% of amino groups. The second polysiloxane has a viscosity of from about 5 mm²s⁻¹ to about 10,000 mm²s⁻¹ at 25°C, such as from about 5 mm²s⁻¹ to about 5,000 mm²s⁻¹, from about 10 mm²s⁻¹ to about 1,000 mm²s⁻¹, or from about 20 mm²s⁻¹ to about 350 mm²s⁻¹. The second polysiloxane has a molecular weight of from about 400 to about 65,000. For example, the molecular weight of the second polysiloxane may range from about 800 to about 50,000, from about 400 to about 30,000, or from about 400 to about 15,000. According to one aspect, the second polysiloxane may be nonionic. According to another aspect, the second polysiloxane may be a linear silicone.

Exemplary second non-volatile polysiloxanes useful herein include polyalkyl or polyaryl siloxanes in accordance with the following the general formula (II): 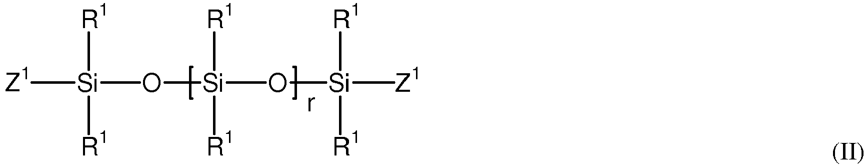 wherein R¹ is alkyl or aryl, and r is an integer from about 7 to about 850, such as from about 7 to about 665, from about 7 to about 400, or from about 7 to about 200. Z¹ represents groups which block the ends of the silicone chains. The alkyl or aryl groups substituted on the siloxane chain (R¹) or at the ends of the siloxane chains Z¹ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. According to an embodiment, suitable Z¹ groups include hydroxy, methyl, methoxy, ethoxy, propoxy, and aryloxy. The two R¹ groups on each silicon atom may represent the same group or different groups. According to one embodiment, the two R¹ groups may represent the same group. Suitable R¹ groups include methyl, ethyl, propyl, phenyl, methylphenyl and phenylmethyl. Exemplary silicone compounds include polydimethylsiloxane, polydiethylsiloxane, and polymethylphenylsiloxane. According to one embodiment, polydimethylsiloxane is the second polysiloxane. Commercially available silicone compounds useful herein include, for example, those available from the General Electric Company in their TSF451 series, and those available from Dow Coming in their Dow Corning SH200 series.

### c. Aminosilicone

The hair care composition of the present invention may comprise an amino silicone having less than about 0.5 wt.% nitrogen by weight of the aminosilicone, such as less than about 0.2 wt.%, or less than about 0.1 wt.%, in view of friction reduction benefit. It has been surprisingly found that higher levels of nitrogen (amine functional groups) in the amino silicone tend to result in less friction reduction, and consequently less conditioning benefit from the aminosilicone. The aminosilicone useful herein may have at least one silicone block with greater than 200 siloxane units, in view of friction reduction benefit. The aminosilicones useful herein include, for example, quaternized aminosilicone and non-quaternized aminosilicone.

In one embodiment, the aminosilicones useful herein are water-insoluble. In the present invention, "water-insoluble amino silicone" means that the aminosilicone has a solubility of 10g or less per 100g water at 25°C, in another embodiment 5g or less per 100g water at 25°C, and in another embodiment 1g or less per 100g water at 25°C. In the present invention, "water-insoluble amino silicone" means that the aminosilicone is substantially free of copolyol groups. If copolyol groups are present, they are present at a level of less than 10 wt.%, less than 1 wt.%, or less than 0.1 wt.% by weight of the amionosilicone.

According to one embodiment, aminosilicone useful herein are those which conform to the general formula (III):

(R²)ₐG₃₋ₐ-Si(-O-SiG₂)ₙ(-O-SiG_{b}(R²)_{2-b})ₘ-O-SiG₃₋ₐ(R²)ₐ (IIII)

wherein G is hydrogen, phenyl, hydroxy, or C₁-C₈ alkyl, such as methyl; a is an integer having a value from 1 to 3, such as 1; b is an integer having a value from 0 to 2, such as 1; n is a number from 1 to 2,000, such as from 100 to 1,800, from 300 to 800, or from 500 to 600; m is an integer having a value from 0 to 1,999, such as from 0 to 10, or 0; R² is a monovalent radical conforming to the general formula C_{q}H_{2q}L, wherein q is an integer having a value from 2 to 8 and L is selected from the following groups: -N(R³₂)CH₂-CH₂-N(R³₂)₂; -N(R³)₂; -N⁺(R³)₃A⁻; -N(R³)CH₂CH₂-N⁺R³H₂A⁻; wherein R³ is hydrogen, phenyl, benzyl, or a saturated hydrocarbon radical, such as an alkyl radical from about C₁ to about C₂₀; A⁻ is a halide ion. According to an embodiment, L is - N(CH₃)₂ or -NH₂. According to another embodiment, L is -NH₂.

The aminosilicone of the above formula is used at levels by weight of the composition of from about 0.1 wt.% to about 5 wt%, alternatively from about 0.2 wt.% to about 2 wt.%, alternatively from about 0.2 wt.% to about 1.0 wt.%, and alternatively from about 0.3 wt.% to about 0.8 wt.%.

According to one embodiment, the aminosilicone may include those compounds corresponding to formula (III) wherein m=0; a=1; q=3; G=methyl; n is from about 1400 to about 1700, such as about 1600; and L is -N(CH₃)₂ or -NH₂, such as -NH₂. According to another embodiment, the aminosilicone may include those compounds corresponding to formula (III) wherein m=0; a=1; q=3; G=methyl; n is from about 400 to about 800, such as from about 500 to around 600; and L is L is -N(CH₃)₂ or -NH₂, such as -NH₂. Accordingly, the aforementioned aminosilicones can be called terminal aminosilicones, as one or both ends of the silicone chain are terminated by nitrogen containing group. Such terminal aminosilicones may provide improved friction reduction compared to graft aminosilicones.

Another example of an aminosilicone useful herein includes, for example, quaternized aminosilicone having a tradename KF8020 available from Shinetsu.

The above aminosilicones, when incorporated into the hair care composition, can be mixed with solvent having a lower viscosity. Such solvents include, for example, polar or non-polar, volatile or non-volatile oils. Such oils include, for example, silicone oils, hydrocarbons, and esters. Among such a variety of solvents, exemplary solvents include those selected from the group consisting of non-polar, volatile hydrocarbons, volatile cyclic silicones, non-volatile linear silicones, and mixtures thereof. The non-volatile linear silicones useful herein are those having a viscosity of from about 1 mm²s⁻¹ to about 20,000 mm²s⁻¹, such as from about 20 mm²s⁻¹ to about 10,000 mm²s⁻¹, at 25°C. According to one embodiment, the solvents are non-polar, volatile hydrocarbons, especially non-polar, volatile isoparaffins, in view of reducing the viscosity of the aminosilicones and providing improved hair conditioning benefits such as reduced friction on dry hair. Such mixtures may have a viscosity of from about 1,000 mPas to about 100,000 mPas, and alternatively from about 5,000 mPas to about 50,000 mPas.

### d. Silicone Copolymer Emulsion

The hair care composition of the present invention may comprise a silicone copolymer emulsion with an internal phase viscosity of greater than about 100 × 10⁶ mm²s⁻¹. The silicone copolymer emulsion may be present in an amount of from about 0.1 wt.% to about 15 wt.%, alternatively from about 0.3 wt.% to about 10 wt%, and alternatively about 0.5 wt.% to about 5 wt.%, by weight of the composition, in view of providing clean feel.

According to one embodiment, the silicone copolymer emulsion has a viscosity at 25°C of greater than about 100 × 10⁶ mm²s⁻¹, alternatively greater than about 120 × 10⁶ mm²s⁻¹, and alternatively greater than about 150 × 10⁶ mm²s⁻¹. According to another embodiment, the silicone copolymer emulsion has a viscosity at 25°C of less than about 1000 × 10⁶ mm²s⁻¹, alternatively less than about 500 × 10⁶ mm²s⁻¹, and alternatively less than about 300 × 10⁶ mm²s⁻¹. To measure the internal phase viscosity of the silicone copolymer emulsion, one may first break the polymer from the emulsion. By way of example, the following procedure can be used to break the polymer from the emulsion: 1) add 10 grams of an emulsion sample to 15 milliliters of isopropyl alcohol; 2) mix well with a spatula; 3) decant the isopropyl alcohol; 4) add 10 milliliters of acetone and knead polymer with spatula; 5) decant the acetone; 6) place polymer in an aluminum container and flatten/dry with a paper towel; and 7) dry for two hours in an 80°C. The polymer can then be tested using any known rheometer, such as, for example, a CarriMed, Haake, or Monsanto rheometer, which operates in the dynamic shear mode. The internal phase viscosity values can be obtained by recording the dynamic viscosity (n') at a 9.900^{∗}10⁻³ Hz frequency point. According to one embodiment, the average particle size of the emulsions is less than about 1 micron, such as less than about 0.7 micron.

The silicone copolymer emulsions of the present invention may comprise a silicone copolymer, at least one surfactant, and water.

The silicone copolymer results from the addition reaction of the following two materials in the presence of a metal containing catalyst:
(i) a polysiloxane with reactive groups on both termini, represented by a general formula (IV): 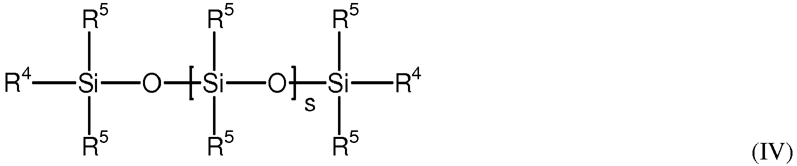 wherein:
   R⁴ is a group capable of reacting by chain addition reaction such as, for example, a hydrogen atom, an aliphatic group with ethylenic unsaturation (i.e., vinyl, allyl, or hexenyl), a hydroxyl group, an alkoxyl group (i.e., methoxy, ethoxy, or propoxy), an acetoxyl group, or an amino or alkylamino group;
   R⁵ is alkyl, cycloalkyl, aryl, or alkylaryl and may include additional functional groups such as ethers, hydroxyls, amines, carboxyls, thiols esters, and sulfonates; in an embodiment, R⁵ is methyl. Optionally, a small mole percentage of the groups may be reactive groups as described above for R⁵, to produce a polymer which is substantially linear but with a small amount of branching. In this case, the level of R⁵ groups equivalent to R⁴ groups may be less than about 10% on a mole percentage basis, such as less than about 2%;
   s is an integer having a value such that the polysiloxane of formula (IV) has a viscosity of from about 1 mm²s⁻¹ to about 1 × 10⁶ mm²s⁻¹;
      and,
(ii) at least one silicone compound or non-silicone compound comprising at least one or at most two groups capable of reacting with the R⁴ groups of the polysiloxane in formula (IV). According to one embodiment, the reactive group is an aliphatic group with ethylenic unsaturation.

The metal-containing catalysts used in the above described reactions are often specific to the particular reaction. Such catalysts are known in the art. Generally, they are materials containing metals such as platinum, rhodium, tin, titanium, copper, lead, etc.

The mixture used to form the emulsion also may contain at least one surfactant. This can include non-ionic surfactants, cationic surfactants, anionic surfactants, alkylpolysaccharides, amphoteric surfactants, and the like. The above surfactants can be used individually or in combination.

An exemplary method of making the silicone copolymer emulsions described herein comprises the steps of 1) mixing materials (a) described above with material (b) described above, followed by mixing in an appropriate metal containing catalyst, such that material (b) is capable of reacting with material (a) in the presence of the metal containing catalyst; 2) further mixing in at least one surfactant and water; and 3) emulsifying the mixture. Methods of making such silicone copolymer emulsions are disclosed in U.S. Pat. No. 6,013,682; PCT Application No. WO 01/58986 A1; and European Patent Application No. EP0874017 A2.

A commercially available example of a silicone copolymer emulsion is an emulsion of about 60-70 wt.% of divinyldimethicone/dimethicone copolymer having an internal phase viscosity of minimum 120 × 10⁶ mm²s⁻¹, available from Dow Coming with a tradename HMW2220.

### e. Silicone polymer containing quaternary groups

The hair care composition of the present invention may comprise a silicone polymer containing quaternary groups (i.e., a quaternized silicone polymer). The quaternized silicone polymer provides improved conditioning benefits such as smooth feel, reduced friction, prevention of hair damage. Especially, the quaternary group can have good affinity with damaged/colorant hairs. The quaternized silicone polymer is present in an amount of from about 0.1 wt.% to about 15 wt.%, based on the total weight of the hair conditioning composition. For example, according to an embodiment, the quaternized silicone polymer may be present in an amount from about 0.2 wt.% to about 10 wt.%, alternatively from about 0.3 wt.% to about 5 wt.%, and alternatively from about 0.5 wt.% to about 4 wt.%, by weight of the composition.

The quaternized silicone polymer of the present invention is comprised of at least one silicone block and at least one non-silicone block containing quaternary nitrogen groups, wherein the number of the non-silicone blocks is one greater than the number of the silicone blocks. The silicone polymers correspond to the general structure (V):

A¹-B-(A²-B)ₘ₋A¹ (V)

wherein, B is a silicone block having greater than 200 siloxane units; A¹ is an end group which may contain quaternary groups; A² is a non-silicone block containing quaternary nitrogen groups; and m is an integer 0 or greater, with the proviso that if m=0 then the A¹ group contains quaternary groups.

Structures corresponding to the general formula, for example, are disclosed in U.S. Pat. No. 4,833,225, in U.S. Patent Application Publication No. 2004/0138400, in U.S. Patent Application Publication No. 2004/0048996, and in U.S. Patent Application Publication No. 2008/0292575.

In one embodiment, the silicone polymers can be represented by the following structure (VI) 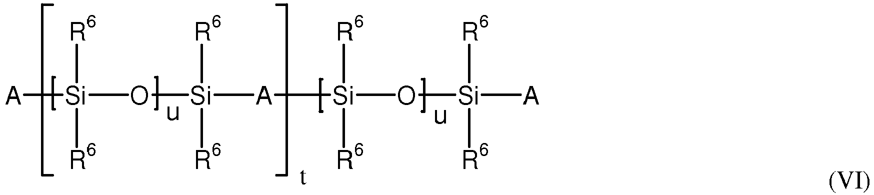 wherein, A is a group which contains at least one quaternary nitrogen group, and which is linked to the silicon atoms of the silicone block by a silicon-carbon bond, each A independently can be the same or different; R⁶ is an alkyl group of from about 1 to about 22 carbon atoms or an aryl group; each R⁶ independently can be the same or different; t is an integer having a value of from 0 or greater, for example t can be less than 20, or less than 10; and u is an integer greater than about 200, such as greater than about 250, or greater than about 300, and u may be less than about 700, or less than about 500. According to an embodiment, R⁶ is methyl.

### f. Grafted Silicone Copolyol

The hair care composition of the present invention may comprise a grafted silicone copolyol in combination with the quaternized silicone polymer. It is believed that this grafted silicone copolyol can improve the spreadability of the quaternized silicone polymer by reducing the viscosity of the quaternized silicone polymer, and also can stabilize the quaternized silicone polymer in aqueous conditioner matrix. It is also believed that, by such improved spreadability, the hair care compositions of the present invention can provide better dry conditioning benefits such as friction reduction and/or prevention of damage with reduced tacky feel. It has been surprisingly found that the combination of the quaternized silicone polymer, grafted silicone copolyol, and cationic surfactant system comprising di-alkyl quaternized ammonium salt cationic surfactants provides improved friction reduction benefit, compared to a similar combination. Such similar combinations are, for example, a combination in which the grafted silicone copolyol is replaced with end-capped silicone copolyol, and another combination in which the cationic surfactant system is substantially free of di-alkyl quaternized ammonium salt cationic surfactants.

The grafted silicone copolyol is contained in the composition at a level such that the weight % of the grafted silicone copolyol to its mixture with quaternized silicone copolymer is in the range of from about 1 wt.% to about 50 wt.%, alternatively from about 5 wt.% to about 40 wt.%, and alternatively from about 10 wt.% to 30 wt.%.

The grafted silicone copolyols useful herein are those having a silicone backbone such as dimethicone backbone and polyoxyalkylene substitutions such as polyethylene oxide or/and polypropylene oxide substitutions. The grafted silicone copolyols useful herein have a hydrophilic-lipophilic balance (HLB) value of from about 5 to about 17, such as from about 8 to about 17, or from about 8 to about 12. The grafted silicone copolyols having the same INCI name have a variety of the weight ratio, depending on the molecular weight of the silicone portion and the number of the polyethylene oxide or/and polypropylene oxide substitutions.

According to an embodiment, exemplary commercially available grafted dimethicone copolyols include, for example: those having a tradename Silsoft 430 having an HLB value of from about 9 to about 12 (INCI name "PEG/PPG-20/23 dimethicone") available from GE; those having a tradename Silsoft 475 having an HLB value of from about 13 to about 17 (INCI name "PEG-23/PPG-6 dimethicone"); those having a tradename Silsoft 880 having an HLB value of from about 13 to about 17 (INCI name "PEG-12 dimethicone"); those having a tradename Silsoft 440 having an HLB value of from about 9 to about 12 (INCI name "PEG-20/PPG-23 dimethicone"); those having a tradename DC5330 (INCI name "PEG-15/PPG-15 dimethicone") available from Dow Corning.

The above quaternized silicone polymer and the grafted silicone copolyol may be mixed and emulsified by an emulsifying surfactant, prior to incorporating them into a gel matrix formed by cationic surfactants and high melting point fatty compounds, as discussed below. It is believed that, this pre-mixture can improve behavior of the quaternized silicone polymer and the grafted silicone copolyol, for example, increase the stability and reduce the viscosity to form more homogenized formulation together with the other components. Such emulsifying surfactant can be used at a level of about 0.001 wt.% to about 1.5 wt.%, alternatively from about 0.005% to about 1.0%, and alternatively from about 0.01 wt.% to about 0.5 wt.%, based on the total weight of the hair conditioning composition. Such surfactants may be nonionic, and have an HLB value of from about 2 to about 15, such as from about 3 to about 14, or from about 3 to about 10. Commercially available examples of emulsifying surfactant include nonionic surfactants having an INCI name C12-C14 Pareth-3 and having an HLB value of about 8 supplied from NIKKO Chemicals Co., Ltd. with tradename NIKKOL BT-3.

According to one embodiment, the hair care composition comprises a combination of two or more silicone conditioning agents, along with an EDDS sequestering agent and a gel matrix.

In one embodiment, the hair care composition comprises a polyalkylsiloxane mixture comprising (i) a first polyalkylsiloxane which is non-volatile, substantially free of amino groups, and has a viscosity of from about 100,000 mm²s⁻¹ to about 30,000,000 mm²s⁻¹, and (ii) a second polyalkylsiloxane which is non-volatile, substantially free of amino groups, and has a viscosity of from about 5 mm²s⁻¹ to about 10,000 mm²s⁻¹; an aminosilicone having less than about 0.5 wt.% nitrogen by weight of the aminosilicone; and a silicone copolymer emulsion with an internal phase viscosity of greater than about 100 × 10⁶ mm²s⁻¹, as measured at 25°C. For example, in another embodiment, the hair care composition comprises from about 0.5 wt.% to about 10 wt.% of a polyalkylsiloxane mixture comprising (i) a first polyalkylsiloxane which is non-volatile, substantially free of amino groups, and has a viscosity of from about 100,000 mm²s⁻¹ to about 30,000,000 mm²s⁻¹, and (ii) a second polyalkylsiloxane which is non-volatile, substantially free of amino groups, and has a viscosity of from about 5 mm²s⁻¹ to about 10,000 mm²s⁻¹; from about 0.1 wt% to about 5 wt.% of an aminosilicone having less than about 0.5 wt.% nitrogen by weight of the aminosilicone; and from about 0.1 wt.% to about 5 wt.% of a silicone copolymer emulsion with an internal phase viscosity of greater than about 100 × 10⁶ mm²s⁻¹, as measured at 25°C.

In another embodiment, the hair care composition comprises a silicone polymer containing quaternary groups wherein said silicone polymer comprises silicone blocks with greater than about 200 siloxane units; and a grafted silicone copolyol. For example, in another embodiment, the hair care composition comprises from about 0.1 wt.% to about 15 wt.% of a silicone polymer containing quaternary groups wherein said silicone polymer comprises silicone blocks with greater than about 200 siloxane units; and a grafted silicone copolyol at a level such that the weight % of the grafted silicone copolyol in its mixture with the quaternized silicone polymer is in the range of from about 1 wt.% to about 50 wt.%.

In yet another embodiment, the hair care composition comprises an aminosilicone having a viscosity of from about 1,000 centistokes to about 1,000,000 centistokes, and less than about 0.5% nitrogen by weight of the aminosilicone; and (2) a silicone copolymer emulsion with an internal phase viscosity of greater than about 120 × 10⁶ centistokes, as measured at 25°C.

### 2. Other Conditioning Agents

Also suitable for use in the hair care compositions herein are the conditioning agents described by the Procter & Gamble Company in U.S. Pat. Nos. 5,674,478, and 5,750,122. Also suitable for use herein are those conditioning agents described in U.S. Pat. Nos. 4,529,586, 4,507,280, 4,663,158, 4,197,865, 4,217, 914, 4,381,919, and 4,422, 853.

### a. Organic Conditioning Oils

The hair care compositions of the present invention may also further comprise an organic conditioning oil. According to embodiments of the present invention, the hair care composition may comprise from about 0.05 wt.% to about 3 wt.%, from about 0.08 wt.% to about 1.5 wt.%, or even from about 0.1 wt% to about 1 wt%, of at least one organic conditioning oil as the conditioning agent, in combination with other conditioning agents, such as the silicones (described herein). Suitable conditioning oils include hydrocarbon oils, polyolefins, and fatty esters. Suitable hydrocarbon oils include, but are not limited to, hydrocarbon oils having at least about 10 carbon atoms, such as cyclic hydrocarbons, straight chain aliphatic hydrocarbons (saturated or unsaturated), and branched-chain aliphatic hydrocarbons (saturated or unsaturated), including polymers and mixtures thereof. Straight-chain hydrocarbon oils are typically from about C12 to about C19. Branched-chain hydrocarbon oils, including hydrocarbon polymers, typically will contain more than 19 carbon atoms. Suitable polyolefins include liquid polyolefins, liquid poly-α-olefins, or even hydrogenated liquid poly-α-olefins. Polyolefins for use herein may be prepared by polymerization of C4 to about C14 or even C6 to about C12. Suitable fatty esters include, but are not limited to, fatty esters having at least 10 carbon atoms. These fatty esters include esters with hydrocarbyl chains derived from fatty acids or alcohols (e.g. mono-esters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.). The fatty esters may be unsaturated, partially hydrogenated or fully hydrogenated.

### 3. Nonionic Polymers

The hair care composition of the present invention may also further comprise a nonionic polymer. According to an embodiment, the conditioning agent for use in the hair care composition of the present invention may include a polyalkylene glycol polymer. For example, polyalkylene glycols having a molecular weight of more than about 1000 are useful herein. Useful are those having the following general formula (VIII): 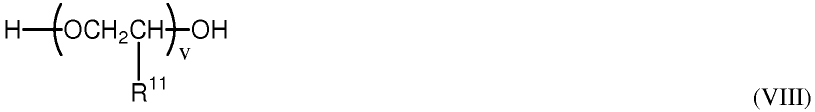 wherein R¹¹ is selected from the group consisting of H, methyl, and mixtures thereof; and v is the number of ethoxy units. The polyalkylene glycols, such as polyethylene glycols, can be included in the hair care compositions of the present invention at a level of from about 0.001 wt.% to about 10 wt.%. In an embodiment, the polyethylene glycol is present in an amount up to about 5 wt.% based on the weight of the composition. Polyethylene glycol polymers useful herein are PEG-2M (also known as Polyox WSR^{®} N-10, which is available from Union Carbide and as PEG-2,000); PEG-5M (also known as Polyox WSR^{®} N-35 and Polyox WSR^{®} N-80, available from Union Carbide and as PEG-5,000 and Polyethylene Glycol 300,000); PEG-7M (also known as Polyox WSR^{®} N-750 available from Union Carbide); PEG-9M (also known as Polyox WSR^{®} N-3333 available from Union Carbide); and PEG-14 M (also known as Polyox WSR^{®} N-3000 available from Union Carbide).

### 4. Suspending Agent

The hair care compositions of the present invention may further comprise a suspending agent at concentrations effective for suspending water-insoluble material in dispersed form in the compositions or for modifying the viscosity of the composition. Such concentrations range from about 0.1 wt.% to about 10 wt.%, or even from about 0.3 wt.% to about 5.0 wt.%.

Suspending agents useful herein include anionic polymers and nonionic polymers. Useful herein are vinyl polymers such as cross linked acrylic acid polymers with the CTFA name Carbomer, cellulose derivatives and modified-cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carrageenan, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Commercially available viscosity modifiers highly useful herein include Carbomers with trade names Carbopol^{®} 934, Carbopol^{®} 940, Carbopol^{®} 950, Carbopol^{®} 980, and Carbopol^{®} 981, all available from B. F. Goodrich Company, acrylates/steareth-20 methacrylate copolymer with trade name ACRYSOL^{™} 22 available from Rohm and Hass, nonoxynyl hydroxyethylcellulose with trade name Amercell^{™} POLYMER HM-1500 available from Amerchol, methylcellulose with trade name BENECEL^{®}, hydroxyethyl cellulose with trade name NATROSOL^{®}, hydroxypropyl cellulose with trade name KLUCEL^{®}, cetyl hydroxyethyl cellulose with trade name POLYSURF^{®} 67, all supplied by Hercules, ethylene oxide and/or propylene oxide based polymers with trade names CARBOWAX^{®} PEGs, POLYOX WASRs, and UCON^{®} FLUIDS, all supplied by Amerchol.

Other optional suspending agents include crystalline suspending agents which can be categorized as acyl derivatives, long chain amine oxides, and mixtures thereof. These suspending agents are described in U.S. Pat. No. 4,741,855.

These suspending agents include ethylene glycol esters of fatty acids in one aspect having from about 16 to about 22 carbon atoms. In one aspect, useful suspending agents include ethylene glycol stearates, both mono and distearate, but in one aspect, the distearate containing less than about 7% of the mono stearate. Other suitable suspending agents include alkanol amides of fatty acids, having from about 16 to about 22 carbon atoms, or even about 16 to 18 carbon atoms, examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin) a commercial example of which is Thixin^{®} R available from Rheox, Inc. Long chain acyl derivatives, ethylene glycol esters of long chain carboxylic acids, long chain amine oxides, and alkanol amides of long chain carboxylic acids in addition to the materials listed above may be used as suspending agents.

Other long chain acyl derivatives suitable for use as suspending agents include N,N-dihydrocarbyl amido benzoic acid and soluble salts thereof (e.g., Na, K), particularly N,N-di(hydrogenated) C16, C18 and tallow amido benzoic acid species of this family, which are commercially available from Stepan Company (Northfield, Ill., USA).

Examples of suitable long chain amine oxides for use as suspending agents include alkyl dimethyl amine oxides, e.g., stearyl dimethyl amine oxide.

Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

### 5. Deposition Aids

The hair care compositions of the present invention may further comprise a deposition aid, such as a cationic polymer. Cationic polymers useful herein are those having an average molecular weight of at least about 5,000, alternatively from about 10,000 to about 10 million, and alternatively from about 100,000 to about 2 million.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol (made by hydrolysis of polyvinyl acetate), maleic anhydride, propylene glycol, and ethylene glycol. Other suitable cationic polymers useful herein include, for example, cationic celluloses, cationic starches, and cationic guar gums.

The cationic polymer can be included in the hair care compositions of the present invention at a level of from about 0.001 wt.% to about 10 wt.%. In one embodiment, the cationic polymer is present in an amount up to about 5 wt% based on the weight of the composition.

### 6. Deposition Polymer

In a further embodiment of the present invention, the composition of the present invention may further comprise a deposition polymer, preferable anionic/acid-deposition polymer. The deposition polymer is included at a level by weight of the composition of, from about 0.03% to about 8%, preferably from about 0.05% to about 3%, more preferably from about 0.1% to about 1%.

It is preferred that the weight ratio of (i) the deposition polymer to (ii) a sum of the monoalkyl amine salt cationic surfactant, di-alkyl quaternized ammonium salt cationic surfactant, and high melting point fatty compound is from about 1:1 to about 1:160, more preferably from about 1:2.5 to about 1:120, still more preferably from about 1:3.5 to about 1:80. If the weight ratio of (i) to (ii) is too low, the composition may provide lower deposition of cationic surfactants, high melting point fatty compounds, and/or silicone compounds. If the weight ratio of (i) to (ii) is too high, the composition may influence rheology, and may undesirably decrease rheology of the composition.

The deposition polymer useful herein is a copolymer comprising: a vinyl monomer (A) with a carboxyl group in the structure; and a vinyl monomer (B) expressed by the following formula (1):

CH₂=C(R¹)-CO-X-(Q-O)ᵣ-R² (1)

wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and
wherein the vinyl monomer (A) is contained at a level of from about 10 mass% to about 50 mass%, and the vinyl monomer (B) is contained at level of from about 50 mass% to about 90 mass%.

### Vinyl Monomer (A)

The copolymer of the present invention contains a vinyl monomer (A) having a carboxyl group in the structure. The copolymer may contain one type of the vinyl monomer (A), or may contain two or more types of the vinyl monomer (A). The vinyl monomer (A) is preferably anionic.

This vinyl monomer (A) is contained at a level of from about 10 mass% based on the total mass of the copolymer, preferably from about 15 mass%, more preferably 20 mass% or higher, and even more preferably 25 mass% or higher, in view of improved deposition of cationic surfactants, fatty compounds and/or silicones, and to about 50 mass%, preferably 45 mass% or less, and more preferably 40 mass% or less, in view of not-deteriorating smoothness during application and/or the product viscosity.

Non-limited example of the vinyl monomer (A) having a carboxyl group include, for example, unsaturated carboxylic acid monomers having 3 to 22 carbon atoms. The unsaturated carboxylic acid monomer has, preferably 4 or more carbon atoms, and preferably 20 or less carbon atoms, more preferably 18 or less carbon atoms, still more preferably 10 or less carbon atoms, and even more preferably 6 or less carbon atoms. Furthermore, the number of carboxyl groups in the vinyl monomer (A) is preferably from 1 to 4, more preferably from 1 to 3, even more preferably from 1 to 2, and most preferably 1.

In view of improved deposition of cationic surfactants, fatty compounds and/or silicones, the vinyl monomer (A) is preferably an unsaturated carboxylic acid monomer expressed by the following formula (2) or formula (3), more preferably those expressed by the formula (2).

CH₂=C(R³)-CO-(O-(CH₂)ₘ-CO)ₙ-OH (2)

wherein: R³ represents a hydrogen atom or a methyl group, preferably a hydrogen atom; m represents an integer of 1 through 4, preferably 2 to 3; and n represents an integer of 0 through 4, preferably 0 to 2, and most preferably 0.

CH₂=C(R⁴)-COO-(CH₂)ₚ-OOC-(CH₂)_{q}-COOH (3)

wherein: R⁴ represents a hydrogen atom or a methyl group, preferably a hydrogen atom; p and q independently represent an integer of 2 through 6, preferably 2 to 3.

Examples of those expressed by the formula (2) include (meth)acrylic acid, crotonic acid, maleic acid, fumaric acid, itaconic acid, angelic acid, tiglic acid, 2-carboxy ethyl acrylate oligomer, and the like. Among them, preferred are acrylic acid and methacrylic acid, and more preferred is acrylic acid. Examples of those expressed by the formula (3) include acryloyloxy ethyl succinate, 2-methacryloyloxy ethyl succinate, and the like.

### Vinyl Monomer (B)

The copolymer contains a vinyl monomer (B). The copolymer may contain one type of the vinyl monomer (B), or may contain two or more types of the vinyl monomer (B). The vinyl monomer (B) is preferably nonionic.

The vinyl monomer (B) is contained at a level of from about 50 mass% based on the total mass of the copolymer in view of improving the feel and the smoothness during application, and to about 90 mass% based on the total mass of the copolymer, preferably to about 85 mass%, more preferably to about 80 mass%, still more preferably 75 mass%, in view of improved deposition of cationic surfactants, fatty compounds and/or silicones.

The Vinyl monomers (B) useful herein are those expressed by formula (4).

CH₂=C(R¹)-CO-X-(Q-O)ᵣR² (4)

wherein: R¹ represents a hydrogen atom or a methyl group; R² represents a hydrogen atom or an alkyl group with 1 through 5 carbon atoms, which may have a substitution group; Q represents an alkylene group with 2 through 4 carbon atoms which may also have a substitution group; r represents an integer from 2 through 15; and X represents an oxygen atom or an NH group; and in the structure - (Q - O)ᵣ - R², the number of atoms bonded in a straight chain is 70 or less.

If R² has a substitution group, the substitution group is a substitution group that does not react with other parts of the copolymer. The vinyl monomer (B) is preferably hydrophilic, and therefore R² is preferably a hydrogen atom or an alkyl group with 1 ~ 3 carbon atoms, and more preferably a hydrogen atom or an alkyl group with 1 or 2 carbon atoms.

X preferably represents an oxygen atom.

Q represents preferably an alkylene group with 2 through 3 carbon atoms which may also have a substitution group, and more preferably an alkylene group with 2 through 3 carbon atoms without any substitution group. If the alkylene group of Q has a substitution group, it is preferred that such substitution group does not react with other parts of the copolymer, more preferably such substitution group has a molecular weight of 50 or less, still more preferably such substitution group has a molecular weight that is smaller than the structural moiety of - (Q - O)ᵣ -. Examples of such substitution group include a hydroxyl group, methoxy group, ethoxy group, and the like.

r represents preferably 3 or higher, and preferably 12 or less, in view of improved deposition of cationic surfactants, fatty compounds and/or silicones, and/or in view of smoothness during application.

As described above, in the structure - (Q - O)ᵣ - R², the number of atoms that are bonded by the straight chain is 70 or less. For example, if Q represents an n-butylene group, r = 15, and R² represents an n-pentyl group, the number of atoms that are bonded in the straight chain of the structure - (Q - O)ᵣ - R² is calculated as 80, which therefore is outside of the scope. The number of atoms bonded in the straight chain in the structure - (Q - O)ᵣ - R² is preferably 60 or less, more preferably 40 or less, even more preferably 28 or less, and particularly preferably 20 or less, in view of improved deposition of cationic surfactants, fatty compounds and/or silicones, and/or in view of smoothness during application.

Examples of the vinyl monomer (B) include, methoxy polyethylene glycol (meth)acrylate (where the number of repetitions of polyethylene glycol (r in formula (4)) is between 2 ~ 15), polyethylene glycol (meth)acrylate (where the number of repetitions of polyethylene glycol (r in formula (4)) is between 2 ~ 15), methoxy polyethylene glycol / polypropylene glycol (meth)acrylate (where the number of repetitions of polyethylene glycol / polypropylene glycol (r in formula (4)) is between 2 ~ 15), polyethylene glycol / polypropylene glycol (meth)acrylate (where the number of repetitions of polyethylene glycol / polypropylene glycol (r in formula (4)) is between 2 ~ 15), methoxy polyethylene glycol / polybutylene glycol (meth)acrylate (where the number of repetitions of polyethylene glycol / polybutylene glycol (r in formula (4)) is between 2 ~ 15), polyethylene glycol / polybutylene glycol (meth)acrylate (where the number of repetitions of polyethylene glycol / polybutylene glycol (r in formula (4)) is between 2 ~ 15), methoxy polyethylene glycol (meth)acrylamide (where the number of repetitions of polyethylene glycol (r in formula (4)) is between 2 ~ 15), and polyethylene glycol (meth)acrylamide (where the number of repetitions of polyethylene glycol (r in formula (4)) is between 2 ~ 15); preferably methoxy polyethylene glycol (meth)acrylate (where the number of repetitions of polyethylene glycol (r in formula (4)) is between 3 ∼ 12), polyethylene glycol (meth)acrylate (where the number of repetitions of polyethylene glycol (r in formula (4)) is between 3 ∼ 12), methoxy polyethylene glycol / polypropylene glycol (meth)acrylate (where the number of repetitions of polyethylene glycol / polypropylene glycol (r in formula (4)) is between 3 ∼ 12), polyethylene glycol / polypropylene glycol (meth)acrylate (where the number of repetitions of polyethylene glycol / polypropylene glycol (r in formula (4)) is between 3 ∼ 12), methoxy polyethylene glycol / polybutylene glycol (meth)acrylate (where the number of repetitions of polyethylene glycol / polybutylene glycol (r in formula (4)) is between 3 ∼ 12), polyethylene glycol / polybutylene glycol (meth)acrylate (where the number of repetitions of polyethylene glycol / polybutylene glycol (r in formula (4)) is between 3 ∼ 12); more preferably methoxy polyethylene glycol (meth)acrylate (where the number of repetitions of polyethylene glycol (r in formula (4)) is between 3 ∼ 12), and polyethylene glycol (meth)acrylate (where the number of repetitions of polyethylene glycol (r in formula (4)) is between 3 ~ 12).

### Vinyl Monomer (C)

In addition to the vinyl monomers (A) and (B), the copolymer may further contain a vinyl monomer (C) having an alkyl group with 12 ~ 22 carbon atoms, in view of providing conditioning effect such as smoothness during application. When included, the amount of the vinyl monomer (C) is preferably 40 mass% or less, more preferably 30 mass% or less, even more preferably 25 mass% or less, and still more preferably 20 mass% or less based on the total mass of the copolymer, in view of improved deposition of cationic surfactants, fatty compounds and/or silicones, and/or in view of smoothness during application.

Preferably, the vinyl monomer (C) is a (meth)acrylate monomer having an alkyl group with 12 - 22 carbon atoms, in view of smoothness during application. Furthermore, vinyl monomers with branched alkyl groups are particularly preferred.

Examples of the (meth)acrylate monomer having an alkyl group with 12 - 22 carbon atoms include myristyl (meth)acrylate, isostearyl (meth)acrylate, stearyl (meth)acrylate, behenyl (meth)acrylate, cetyl (meth)acrylate, lauryl (meth)acrylate, synthetic lauryl (meth)acrylate, (however "synthetic lauryl (meth)acrylate" refers to an alkyl (meth)acrylate having alkyl groups with 12 carbon atoms and alkyl groups with 13 carbon atoms), and the like. Of these, (meth)acrylate monomers having an alkyl group with 12 - 20 carbon atoms are preferable, and (meth)acrylate monomers having an alkyl group with 16 - 18 carbon atoms are more preferable.

The copolymer may contain one type of the vinyl monomer (C), or may contain two or more types of the vinyl monomer (C).

### Other Monomers

In addition to the aforementioned vinyl monomers (A), (B), and (C), the copolymer may also contain other vinyl monomers, to the extent not to deteriorate the effect of the copolymer. Examples of other vinyl monomers include nonionic monomers, amphoteric monomers, semi-polar monomers, cationic monomers, as well as monomers containing a polysiloxane group., preferably nonionic monomers with or without polysiloxane group These other monomers are different from any of the aforementioned vinyl monomers (A), (B), and (C).

Normally the amount of such other monomers, if included, is 40 mass% or less of the total mass of the copolymer, preferably 30 mass% or less, more preferably 20 mass% or less, and even more preferably 10 mass% or less.

In view of improved deposition of cationic surfactants, fatty compounds, and/or silicones, the amount of cationic functional groups in the copolymer is preferably low, and for example cationic functional groups preferably account for 10 mole % or less of all functional groups in the copolymer. More preferably, the copolymer is free of cationic functional groups.

Examples of nonionic monomers include esters of (meth)acrylic acid and alcohols with 1 ~ 22 carbon atoms, amides of (meth)acrylic acid and alkyl amines with 1 ~ 22 carbon atoms, monoesters of (meth)acrylic acid and ethylene glycol, 1,3-propylene glycol or the like, as well as esters where the hydroxyl group of the monoester has been etherified by methanol, ethanol or the like, (meth)acryloyl morpholine and the like.

Examples of amphoteric monomers include (meth)acryl esters having a betaine group, (meth)acrylamide having a betaine group and the like.

Examples of semipolar monomers include (meth)acrylate esters having an amine oxide group, (meth)acrylamides having an amine oxide group, and the like.

Examples of cationic monomers include (meth)acrylate esters having a quaternary ammonium group, (meth)acrylamides having a quaternary ammonium group and the like.

The monomer containing a polysiloxane group is a monomer having a polysiloxane structure and also having a structure that can bond by covalent bond to the copolymer. These component units have high affinity towards silicone oil that is normally used in conjunction in cosmetic material compositions, and are thought to act by bonding the silicone oil to the other component units in the copolymer and thus increasing the adsorption force of silicone oil to the skin and hair, particularly damaged hair.

The polysiloxane structure is a structure where two or more repeating structural units expressed by the following formula (4) are linked.

-(SiR⁵R⁶-O)- (4)

In formula (4), R⁵ and R⁶ independently represent an alkyl group with 1 to 3 carbon atoms or a phenyl group.

The structure that can link via covalent bond to the copolymer can be a structure that has a vinyl structure such as a (meth)acrylate ester, or (meth)acrylamide and that can copolymerize with another monomer, a structure that has a functional group such as a thiol, that can link to the copolymer by chain transfer during polymerization, or a structure that has an isocyanate group, carboxylic acid group, hydroxyl group, amino group, or the like, and that can react and link to the functional groups on the copolymer, but there is no restriction to these structures.

A plurality of these linkable structures can be present in one monomer containing a polysiloxane group. In the copolymer, the polysiloxane structure can link by a graft structure to the main chain, or conversely the polysiloxane structure can be the main chain with the other structure link by a graft structure, and in addition the polysiloxane structure and the other structure can be linked in a straight chain condition by a block structure.

The monomer containing a polysiloxane group is preferably expressed by the following formula (5).

CH₂=C(R⁷)-Z-(SiR⁸R⁹-O)ₛ-R¹⁰ (5)

In the formula, R⁷ represents a hydrogen atom or a methyl group, R⁸ and R⁹ independently represent an alkyl group with 1 to 3 carbon atoms or a phenyl group, R¹⁰ represents an alkyl group with 1 to 8 carbon atoms, Z represents a bivalent linking group or a direct bond, and s represents an integer between 2 to 200.

More preferably, s is 3 or higher, and even more preferably, s is 5 or higher, in view of increased affinity to silicone oil, and preferably s is 50 or less, in view of enhanced copolymerization with the other monomers.

Z represents a bivalent linking group or a direct bond, but a linking group containing one or a combination of two or more of the structures suggested below is preferable. The numbers that are combined is not particularly restricted, but normally is 5 or less. Furthermore, the direction of the following structures are arbitrary (the polysiloxane group side can be on either end). Note, in the following, R represents an alkylene group with 1 to 6 carbon atoms or a phenylene group.

-COO-R-

-CONH-R-

-O-R-

-R-

The monomer expressed by the aforementioned formula (5), include, for example, α-(vinyl phenyl) polydimethyl siloxane, α-(vinyl benzyloxy propyl) polydimethyl siloxane, α-(vinyl benzyl) polymethyl phenyl siloxane, α-(methacryloyl oxypropyl) polydimethyl siloxane, α-(methacryloyloxy propyl) polymethyl phenyl siloxane, α-(methacryloyl amino propyl) polydimethyl siloxane and the like. The monomer containing a polysiloxane group can be a single type, or can be two or more types used in combination.

In order to adjust the molecular weight and the viscosity of the copolymer, a cross-linking agent such as a polyfunctional acrylate or the like can be introduced to the copolymer. However, in this invention, it is preferred that a cross-linking agent is not included in the copolymer.

### Structure Analysis

The amount of the vinyl monomers (A), (B), and (C) as well as other monomers in the copolymer can be measured using IR absorption or Raman scattering by the carbonyl groups, amide bonds, polysiloxane structures, various types of functional groups, carbon backbone and the like, by ¹H-NMR of methyl groups in the polydimethyl siloxane, amide bond sites, and methyl groups and methylene groups adjacent thereto, as well as various types of NMR represented by ¹³C-NMR and the like.

### Weighted Average Molecular Weight

The weighted average molecular weight of the copolymer is preferably 3,000 or higher, more preferably 5,000 or higher, and even more preferably 10,000 or higher, in view of providing conditioning effect via foaming a complex with cationic surfactant, and preferably to about 2,000,000, more preferably 1,000,000 or less, still more preferably 500,000 or less, even more preferably 100,000 or less, and most preferably 50,000 or less, in view of feeling after drying.

The weighted average molecular weight of the copolymer can be measured by gel permeation chromatography (GPC). The development solvent that is used in gel permeation chromatography is not particularly restricted so long as being a normally used solvent, but for example, the measurement can be performed using a solvent blend of water / methanol / acetic acid / sodium acetate.

### Viscosity

The copolymer preferably has a viscosity for a 50 mass% of an aqueous carrier solution of lower alkyl alcohols and polyhydric alcohols, preferably ethanol aqueous solution, more preferably butanediol aqueous solution at 25°C of 5 mPa•s or higher and 50,000 mPa•s or less. The viscosity is more preferably 10 mPa•s or higher, even more preferably 15 mPa•s or higher, but on the other hand is more preferably 10,000 mPa•s or less, and even more preferably 5,000 mPa•s or less. The viscosity of the copolymer is preferably 5 mPa•s or higher and 50,000 mPa•s or less, from the perspective of handling. The viscosity can be measured using a BL-type viscometer.

Similar to the weighted average molecular weight, the viscosity of the copolymer can be adjusted by controlling the degree of polymerization of the copolymer, and can be controlled by increasing or decreasing the amount of a cross-linking agent such as a polyfunctional acrylate or the like that is added.

### 7. Benefit Agents

In an embodiment, the hair care composition further comprises one or more additional benefit agents. The benefit agents comprise a material selected from the group consisting of anti-dandruff agents, vitamins, lipid soluble vitamins, chelants, perfumes, brighteners, enzymes, sensates, attractants, anti-bacterial agents, dyes, pigments, bleaches, and mixtures thereof.

In one aspect said benefit agent may comprise an anti-dandruff agent. Such anti-dandruff particulate should be physically and chemically compatible with the components of the composition, and should not otherwise unduly impair product stability, aesthetics or performance.

According to an embodiment, the hair care composition comprises an anti-dandruff active, which may be an anti-dandruff active particulate. In an embodiment, the anti-dandruff active is selected from the group consisting of: pyridinethione salts; azoles, such as ketoconazole, econazole, and elubiol; selenium sulphide; particulate sulfur; keratolytic agents such as salicylic acid; and mixtures thereof. In an embodiment, the anti-dandruff particulate is a pyridinethione salt.

Pyridinethione particulates are suitable particulate anti-dandruff actives. In an embodiment, the anti-dandruff active is a 1-hydroxy-2-pyridinethione salt and is in particulate form. In an embodiment, the concentration of pyridinethione anti-dandruff particulate ranges from about 0.01 wt.% to about 5 wt.%, or from about 0.1 wt.% to about 3 wt.%, or from about 0.1 wt.% to about 2 wt.%. In an embodiment, the pyridinethione salts are those formed from heavy metals such as zinc, tin, cadmium, magnesium, aluminium and zirconium, generally zinc, typically the zinc salt of 1-hydroxy-2-pyridinethione (known as "zinc pyridinethione" or "ZPT"), commonly 1-hydroxy-2-pyridinethione salts in platelet particle form. In an embodiment, the 1-hydroxy-2-pyridinethione salts in platelet particle form have an average particle size of up to about 20 microns, or up to about 5 microns, or up to about 2.5 microns. Salts formed from other cations, such as sodium, may also be suitable. Pyridinethione anti-dandruff actives are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

In an embodiment, in addition to the anti-dandruff active selected from polyvalent metal salts of pyrithione, the composition further comprises one or more anti-fungal and/or anti-microbial actives. In an embodiment, the anti-microbial active is selected from the group consisting of: coal tar, sulfur, fcharcoal, whitfield's ointment, castellani's paint, aluminum chloride, gentian violet, octopirox (piroctone olamine), ciclopirox olamine, undecylenic acid and its metal salts, potassium permanganate, selenium sulphide, sodium thiosulfate, propylene glycol, oil of bitter orange, urea preparations, griseofulvin, 8-hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone, and azoles, and mixtures thereof. In an embodiment, the anti-microbial is selected from the group consisting of: itraconazole, ketoconazole, selenium sulphide, coal tar, and mixtures thereof.

In an embodiment, the azole anti-microbials is an imidazole selected from the group consisting of: benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and mixtures thereof, or the azole anti-microbials is a triazole selected from the group consisting of: terconazole, itraconazole, and mixtures thereof. When present in the hair care composition, the azole anti-microbial active is included in an amount of from about 0.01 wt.% to about 5 wt.%, or from about 0.1 wt.% to about 3 wt.%, or from about 0.3 wt.% to about 2 wt.%. In an embodiment, the azole anti-microbial active is ketoconazole. In an embodiment, the sole anti-microbial active is ketoconazole.

Embodiments of the hair care composition may also comprise a combination of anti-microbial actives. In an embodiment, the combination of anti-microbial active is selected from the group of combinations consisting of: octopirox and zinc pyrithione, pine tar and sulfur, salicylic acid and zinc pyrithione, salicylic acid and elubiol, zinc pyrithione and elubiol, zinc pyrithione and climbasole, octopirox and climbasole, salicylic acid and octopirox, and mixtures thereof.

In an embodiment, the composition comprises an effective amount of a zinc-containing layered material. In an embodiment, the composition comprises from about 0.001 wt.% to about 10 wt.%, or from about 0.01 wt.% to about 7 wt.%, or from about 0.1 wt.% to about 5 wt.% of a zinc-containing layered material, by total weight of the composition.

Zinc-containing layered materials may be those with crystal growth primarily occurring in two dimensions. It is conventional to describe layer structures as not only those in which all the atoms are incorporated in well-defined layers, but also those in which there are ions or molecules between the layers, called gallery ions (A.F. Wells "Structural Inorganic Chemistry" Clarendon Press, 1975). Zinc-containing layered materials (ZLMs) may have zinc incorporated in the layers and/or be components of the gallery ions. The following classes of ZLMs represent relatively common examples of the general category and are not intended to be limiting as to the broader scope of materials which fit this definition.

Many ZLMs occur naturally as minerals. In an embodiment, the ZLM is selected from the group consisting of: hydrozincite (zinc carbonate hydroxide), aurichalcite (zinc copper carbonate hydroxide), rosasite (copper zinc carbonate hydroxide), and mixtures thereof. Related minerals that are zinc-containing may also be included in the composition. Natural ZLMs can also occur wherein anionic layer species such as clay-type minerals (e.g., phyllosilicates) contain ion-exchanged zinc gallery ions. All of these natural materials can also be obtained synthetically or formed *in situ* in a composition or during a production process.

Another common class of ZLMs, which are often, but not always, synthetic, is layered double hydroxides. In an embodiment, the ZLM is a layered double hydroxide conforming to the formula [M²⁺₁₋ₓM³⁺ₓ(OH)_{2]}^{x+} A^{m-}_{x/m}·nH₂O wherein some or all of the divalent ions (M²⁺) are zinc ions (Crepaldi, EL, Pava, PC, Tronto, J, Valim, JB J. Colloid Interfac. Sci. 2002, 248, 429-42).

Yet another class of ZLMs can be prepared called hydroxy double salts (Morioka, H., Tagaya, H., Karasu, M, Kadokawa, J, Chiba, K Inorg. Chem. 1999, 38, 4211-6). In an embodiment, the ZLM is a hydroxy double salt conforming to the formula [M²⁺₁₋ₓM²⁺₁₊ₓ(OH)_{3(1-y)}]⁺ Aⁿ⁻_{(1=3y)/n}·nH₂O where the two metal ions (M²⁺) may be the same or different. If they are the same and represented by zinc, the formula simplifies to [Zn₁₊ₓ(OH)₂]^{2x+} 2x A⁻·nH₂O). This latter formula represents (where x=0.4) materials such as zinc hydroxychloride and zinc hydroxynitrate. In an embodiment, the ZLM is zinc hydroxychloride and/or zinc hydroxynitrate. These are related to hydrozincite as well wherein a divalent anion replace the monovalent anion. These materials can also be formed *in situ* in a composition or in or during a production process.

In embodiments having a zinc-containing layered material and a pyrithione or polyvalent metal salt of pyrithione, the ratio of zinc-containing layered material to pyrithione or a polyvalent metal salt of pyrithione is from about 5:100 to about 10:1, or from about 2:10 to about 5:1, or from about 1:2 to about 3:1.

The on-scalp deposition of the anti-dandruff active is at least about 1 microgram/cm². The on-scalp deposition of the anti-dandruff active is important in view of ensuring that the anti-dandruff active reaches the scalp where it is able to perform its function. In an embodiment, the deposition of the anti-dandruff active on the scalp is at least about 1.5 microgram/cm², or at least about 2.5 microgram/cm², or at least about 3 microgram/cm², or at least about 4 microgram/cm², or at least about 6 microgram/cm², or at least about 7 microgram/cm², or at least about 8 microgram/cm², or at least about 8 microgram/cm², or at least about 10 microgram/cm². The on-scalp deposition of the anti-dandruff active is measured by having the hair of individuals washed with a composition comprising an anti-dandruff active, for example a composition pursuant to the present invention, by trained a cosmetician according to a conventional washing protocol. The hair is then parted on an area of the scalp to allow an open-ended glass cylinder to be held on the surface while an aliquot of an extraction solution is added and agitated prior to recovery and analytical determination of anti-dandruff active content by conventional methodology, such as HPLC.

### TEST METHODS

It is understood that the test methods that are disclosed in the Test Methods Section of the present application should be used to determine the respective values of the parameters of Applicants' invention as such invention is described and claimed herein.

### A. Emulsion particle size method

This method is designed to measure the oil/lipid particle sizes in emulsion. It is an example of particle size measuring methodology. Other known particle size method may also be used. The Horiba LA-910 and LA-950 instruments use the principle of low-angle Fraunhofer diffraction and Light Scattering from the particles as the means to size particles in a dilution solution.

The emulsion sample is introduced into the Horiba sampling cup, which contain a dilute dispersant solution. The sample is agitated in the sample cup and circulated through the flow cell. During the experiment, light from a laser and lamp are directed through the sample in the flow cell. The light from the laser and lamp diffracts and scatters off the particles and is detected by a seriew of detectors. The scattering and diffraction information travels from the detector to the computer, which then calculates the particle size distribution in the sample.

150 mL1% sodium dodecyl sulfate (SDS) solution is added into a 400 mL beaker. About 0.5 (+/-) g of emulsion is weighed into the beaker. The sample is vigorously with a stir bar on a magnetic stirring plate for 5 minutes. The sample is ready for Particle Size Analysis by Horiba. Samples are analyzed within 10 minutes after sample preparation.

### Horiba LA-910 Laser scattering particle size distribution analyzer:

The appropriate measurement conditions are manually selected as listed below. The Horiba Cup is filled with 150 ml of 0.1% SDS using a measuring cylinder, then Sonicated, Circulated
and Agitated through the cell. If the cell looks clean and background reading looks flat, a blank is run by pressing BLANK. The dispersed sample is added slowly with a disposable pipette to the Horiba cup while the dispersant solution is agitating and circulating through the Horiba system. Do not leave any sample in the syringe and the whole amount of the syringe is always added into the Horiba cup. The sample is added *continuously* and slowly until the %T of the Lamp is 90 ± 2 %. The sample is allowed to agitate and to circulate through the cell for 3 minutes, then press MEASURE to analyze the sample. Once the sample is analyzed, The cell is drained and cleaned with deionized water.

The Graph page is printed and the description of results is shown as D (50), also called the median, that is the particle size at which 50% of the particles are that size or smaller. D (20) and D (90) can also be generated if needed.

The particle size of oils and lipids in personal care composition is often difficult to measure with Particle Size Analyzers like Horiba L910. The particle sizes in the compositions are estimated via optical microscopy.

### Wet and Dry Sensory Hair Feel Evaluation of Rinse-off Conditioner

For the wet feel determination of hair that are treated with rinse-off conditioner products, hair switches of moderately damaged brown European hair are used. Each hair switch weighs 4 g and the length of each hair fiber is 8 inches. The switches are examined via Scanning Electron Microscope before their use in order to verify uniform cuticle direction and the appropriate level of damage.

The water source used for this test must be of low hardness (0-3 grains per gallon). The applied flow during rinsing is 7570 ml per minute at room temperature. The switches are hanged over the sink, combed to detangle and wetted with water for 30 seconds with milking action. After wetting, the excess water is removed via squeezing between the index finger and the middle finger and 0.1 mL of the hair care product is dispensed to the finger of the panelist. A quantity of 0.4 mL of the product is also dispensed on the upper part of the hair switch. The look and feel of the product is evaluated at this stage. Then, then the product is applied with a milking action on the hair for 60 seconds. The detangling, ease of distribution, penetration and feel are evaluated at this stage. Then, the hair switch is rinsed for 30 seconds with milking action and the feel and ease of rinsing is evaluated. The rinsing cycle is repeated once more and the same evaluations are repeated. Then, the hair switch is evaluated for suppleness, combability, and feel. Then the hair is allowed to air dry overnight at a controlled temperature and humidity environment (21°C and 45% Relative Humidity). It is evaluated again for combability, feel and look.

All evaluations are performed by trained and qualified sensory panelists. The same type of hair evaluations are repeated against standard products and the final rating in the scale of 0 to 100 is the result of the various evaluations against hair treated with these standard products of known performance.

### A. Wet and Dry Conditioning Test Method

This test method is designed to allow for a subjective evaluation of the basic performance of rinse-off conditioners for both wet combing and dry combing efficacy. In a typical test, 3 to 5 separate formulations may be assessed for their performance. The assessment may include control treatments containing no silicone and an elevated silicone level to facilitate differentiation of performance. The substrate is virgin brown hair obtainable from a variety of sources that is screened to insure uniformity and lack of meaningful surface damage or low lift bleach damaged hair.

### Treatment Procedure

Four to five 4 gram, 8 inch length switches are combined in a hair switch holder, wet for ten seconds with manipulation with 40°C water of medium hardness (9-10 gpg) to ensure complete and even wetting. The switch is deliquored lightly and Clarifying shampoo is applied uniformly over the length of the combined switches from one inch below the holder towards the tip at a level of 0.1 gram product per one gram of dry hair (0.1 g/g of hair or 2 g for 20 g hair). The switch combo is lathered for 30 seconds by a rubbing motion typical of that used by consumers and rinsed with 40°C water flowing at 1.5 gal/min (with the hair being manipulated) for a further 30 seconds to ensure completeness. This step is repeated. The conditioner treatments are applied in the same way as shampoo above (0.1 g/g of hair or reduced to 0.05 g/g of hair for more concentrated prototypes), milked throughout the switch combo for 30 seconds, left to sit for a further 30 seconds, and rinsed thoroughly with manipulation, again for 30 seconds. The switches are deliquored lightly, separated from each other, hung on a rack so that they are not in contact, and detangled with a wide tooth comb.

### Grading Procedures

For wet combing evaluations using trained graders, the switches are separated on the rack into the five sets with one switch from each treatment included in the grading set. Only two combing evaluations are performed on each switch. The graders are asked to compare the treatments by combing with a narrow tooth nylon comb typical of those used by consumers and rate the ease/difficulty on a zero to ten scale. Ten separate evaluations are collected and the results analyzed by a statistical analysis package for establishing statistical significance. Statistical significance in differences between treatments is determined using Statgraphics Plus 5.1.

For dry combing evaluations, the switches from above are moved into a controlled temperature and humidity room (22°C/50% RH) and allowed to dry overnight. They remain separated as above and panelists are requested to evaluate dry conditioning performance by making three assessments; dry combing ease of the middle of the switch, dry combing ease of the tips, and a tactile assessment of tip feel. The same ten point scale is used for these comparisons. Again, only two panelists make an assessment of each switch set. Statistical analysis to separate differences is performed using the same method as above.

### EXAMPLES

The following examples illustrate the present invention. The examples that do not fall within the definition of claim 1 are comparative examples. The exemplified compositions can be prepared by conventional formulation and mixing techniques. It will be appreciated that other modifications of the present invention within the skill of those in the hair care formulation art can be undertaken without departing from the scope of this invention. All parts, percentages, and ratios herein are by weight unless otherwise specified. Some components may come from suppliers as dilute solutions. The amount stated reflects the weight percent of the active material, unless otherwise specified.

**Table 1: Emulsion Examples**

| Ingredient | | Ex. 1 | Ex. 2 | Ex. 3 | Ex. 4 | Ex. 6 | Ex. 7 | Ex. 8 | Ex. 9 | Ex. 10 |
|---|---|---|---|---|---|---|---|---|---|---|
| Distilled Water | | q.s. | q.s. | q.s. | q.s. | q.s. | q.s. | q.s. | q. s. | |
| Polysorbate ¹ | | 1.73 | 1.91 | 10 | 0.66 | -- | -- | 1.73 | 1.73 | -- |
| Sorbitan Ester ² | | 0.77 | -- | -- | -- | -- | -- | 0.77 | 0.77 | -- |
| Sodium Laureth-3 Sulfate ³ | | -- | -- | -- | -- | 5.0 | -- | -- | -- | -- |
| Monoglyceride ⁴ | | -- | 0.97 | -- | 0.34 | -- | -- | -- | -- | -- |
| Cocoamidopropyl Betaine ⁵ | | -- | -- | -- | -- | -- | 5.0 | -- | -- | -- |
| Soy Oligomer ⁶ | | -- | 10.0 | -- | -- | -- | -- | -- | -- | -- |
| Soy Oligomer Blend ⁷ | | 20.0 | -- | -- | 20.0 | 20.0 | 20.0 | -- | -- | 20 |
| Sefose ⁸ | | -- | -- | 20.0 | -- | -- | -- | -- | -- | -- |
| Glycerine ⁹ | | -- | 7.5 | 50.0 | 45.0 | -- | -- | -- | -- | 50 |
| Ethylene glycol ¹⁰ | | 6.0 | -- | -- | -- | -- | -- | 6.0 | 6.0 | -- |
| Soybean oil¹¹ | | -- | -- | -- | -- | -- | -- | 20.0 | -- | -- |
| Hydrogenated soybean oil¹² | | -- | -- | -- | -- | -- | -- | -- | 20.0 | -- |
| C₁₂₋₁₄ pareth-9¹³ | | -- | -- | -- | -- | -- | -- | - | -- | 2.1 |
| Stearamidopropyl dimethylamine¹⁴ | | -- | -- | -- | -- | -- | -- | -- | -- | 0.4 |
| Preservatives, pH, viscosity adjustment | | Up to 3% | Up to 3% | Up to 3% | Up to 3% | Up to 3% | Up to 3% | Up to 3% | Up to 3% | Up to 3% |
| | | | | | | | | | | |
| **Median Particle Size, nm** | | **202** | **676** | **91** | **443** | **296** | **339** | **119** | **487** | **238** |
| | | | | | | | | | | |
| ¹ Tween 20, from Lonza | | | | | | | | | | |
| ² Span 60, from Croda | | | | | | | | | | |
| ³ Sodium Laureth 3 Sulfate (28% active), from P&G | | | | | | | | | | |
| ⁴ Glyceryle monooleate, from BASF | | | | | | | | | | |
| ⁵ Amphosol HCA-B, from Stepan | | | | | | | | | | |
| ⁶ HY-3050, from Dow Corning | | | | | | | | | | |
| ⁷ HY-3051, from Dow Corning | | | | | | | | | | |
| ⁸ Sefose 1618S from P&G | | | | | | | | | | |
| ⁹ Glycerin USP | | | | | | | | | | |
| ¹⁰ Ethylene glycol from Aldrich | | | | | | | | | | |
| ¹¹Soybean oil, from Cargill | | | | | | | | | | |
| ¹²Soy-125 soy wax, from Candlewic Co. | | | | | | | | | | |
| ¹³ BT9 from Nikkol | | | | | | | | | | |
| ¹⁴ Lexamine S-13 from Inolex Chemcial Co | | | | | | | | | | |

**Table 2. Rinse-Off Conditioner Examples**

| INCI Name | Comparative | Inventive | Inventive |
|---|---|---|---|
| Stearamidopropyl Dimethylammonium¹ | 1.06 | 1.06 | 1.06 |
| Dicetyldimonium Chloride Propylene Glycol² | 0.50 | 0.50 | 0.50 |
| Soy oligomer Emulsion (Ex.4) | -- | 2.5 | 5.0 |
| Soy oligomer³ | 0.50 | -- | -- |
| 1-Hexadecanol | 1.57 | 1.57 | 1.57 |
| 1-Octadecanol⁵ | 2.82 | 2.82 | 2.82 |
| Preservatives, pH, viscosity adjustment | Up to 5% | Up to 5% | Up to 5% |
| Fragrance | 0.50 | 0.50 | 0.50 |
| DI water | q.s. | q.s. | q.s. |
| | | | |
| Wet combing | 5.9 | **7.6** | **6.2** |
| Dry combing | 7.4 | **8.0** | **8.4** |
| Less coated wet feel: change from chassis | 1.87 | **4.38** | |
| Width change from chassis control (dry volume) | 1.25 | **3.13** | |

| | | | |
|---|---|---|---|
| ¹ LEXAMINE S-13, from BAFT, ² Varisoft 432 PPG, from Evonik Degussa Corp. ³ HY-3051, from Dow Corning ⁵ Cetyl alcohol, from P&G ⁵ Stearyl alcohol, from P&G S: statistically different from comparative example | | | |

**Table 3.**

| Ingredients | Control without Soy Oligomer Blend Emulsion Exp 11 | Invention Exp 12 | Control |
|---|---|---|---|
| Citric Acic | - | - | 0.130 |
| Stearamidopropyldimethylamine | - | - | 1.000 |
| Behentrimonium Methosulfate | 2.282 | 2.282 | - |
| Isopropyl Alcohol | 0.570 | 0.570 | - |
| DTDMAC¹ (Quaternium-18) | - | - | 0.750 |
| Hydroxypropyl Guar (Jaguar HP-105) | - | - | 0.350 |
| Cetyl Alcohol | 1.012 | 1.012 | 1.200 |
| Stearyl Alcohol | 5.525 | 5.525 | 0.800 |
| Emulsifying Wax NF (Polawax NF) | - | - | 0.500 |
| Glyceryl Monostearate | - | - | 0.250 |
| Oleyl Alcohol | - | - | 0.250 |
| Terminal Amodimethicone² | - | - | 0.500 |
| Deposition Aid polymer solution (50% aqueous solution)³ | 1.000 | 1.000 | - |
| Emulsion from Example 4 | - | 0.500 | - |
| Disodium EDTA | 0.127 | 0.127 | - |
| Ethylene Diamine Tetraacetic Acid | - | - | 0.100 |
| Benzyl Alcohol | 0.400 | 0.400 | 0.400 |
| Kathon CG | 0.033 | 0.033 | 0.033 |
| Panthenyl Ethyl Ether | 0.030 | 0.030 | 0.030 |
| DL-Panthenol (56% in water) | 0.536 | 0.536 | 0.536 |
| Fragrance | 0.500 | 0.500 | 0.500 |
| Water | q.s. | q.s. | q.s. |
| | | | |
| ¹. Ditallowdimethylammonium chloride | | | |
| ². Terminal amodimethicone is available from Momentive Performance Materials, having a viscosity of about 10,000 cP at 25 ° C. | | | |
| ³. A-931 from Mitsubishi Chemical | | | |

**Table 4.**

| | Formula (see Table 3) | Evaluation Ease of Rinsing* - Rating | Wet Feel |
|---|---|---|---|
| Control | Control | Quickest- 3 | Lower coated/conditioning feel |
| Control without Soy Oligomer Blend Emulsion | Exp 11 | Takes longer to rinse- 1 | Higher coated/conditioning feel. |
| | Conditioner with Depostion Aid and no soy oligomer | | |
| Invention | Exp 12 | Quick rinsing - 2 | Residual feel |
| | Conditioner with Deposition Aid Polymer⁺ + soy oligomer | | |

| | | | |
|---|---|---|---|
| *: Conditioner is applied to a hair switch, spread, milked, and then rinsed off. The feel of rinse is evaluated as below and the averages of the evaluation by 4 panelists is measured. 3 Rinses off very quickly 2 Rinses off quickly 1 Requires longer rinsing | | | |

Residual feel is the negative feel that consumers describe as "feel of chemical left on the hair". Consumers do not relate to high conditioning.

Coated feel is the feel that consumers associate with higher hair conditioning.

From the results above (comparison of Exp 11 and Exp 12), by combining both Deposition Aid polymer and a preemulsified emulsion of soy oligomer blend we can achieve the synergy of the technologies, which enables to condition hair with less residual feel that rinses faster than the corresponding composition containing Deposition Aid polymer and no soy oligomer blend.

Comparison of Exp 12 with control, indicates that hair conditioner formulation containing Deposition Aid polymer and a preemulsified emulsion of soy oligomer blend provides higher coating wet feel than that of the control conditioner, which consumers associate with higher hair conditioning.

The hair care compositions of the present invention may be presented in typical hair care formulations. They may be in the form of solutions, dispersion, emulsions, powders, talcs, encapsulated spheres, spongers, solid dosage forms, foams, and other delivery mechanisms. The compositions of the embodiments of the present invention may be hair tonics, leave-on hair products such as treatment and styling products, rinse-off hair products such as shampoos and conditioners, and any other form that may be applied to hair.

According to one embodiment, the hair care compositions may be provided in the form of a porous, dissolvable solid structure, such as those disclosed in U.S. Patent Application Publication Nos. 2009/0232873; and 2010/0179083.

The hair care compositions are generally prepared by conventional methods such as those known in the art of making the compositions. Such methods typically involve mixing of the ingredients in one or more steps to a relatively uniform state, with or without heating, cooling, application of vacuum, and the like. The compositions are prepared such as to optimize stability (physical stability, chemical stability, photostability) and/or delivery of the active materials. The hair care composition may be in a single phase or a single product, or the hair care composition may be in a separate phases or separate products. If two products are used, the products may be used together, at the same time or sequentially. Sequential use may occur in a short period of time, such as immediately after the use of one product, or it may occur over a period of hours or days.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A hair care composition comprising:
a. from 0.25% to 80% of a pre-emulsified emulsion comprising from 0.005% to 80% of one or more materials selected from the group consisting of metathesized unsaturated polyol esters, with a molecular weight greater than or equal to 1500, by weight of said hair care composition;
wherein said metathesized unsaturated polyol esters are oligomers, wherein said one or more oligomers is a triglyceride oligomer, wherein the triglyceride oligomer is a soy oligomer; wherein an emulsifier is selected from the group consisting of anionic emulsifier, non-ionic emulsifier, cationic emulsifier, amphoteric emulsifier, and mixtures thereof; wherein the average particle size of the pre-emulsified oil in water emulsion is from 20 nanometers to 20 microns; and
b. a cationic surfactant system;
further wherein the composition is stable with respect to one of the following measures selected from emulsion particle size, viscosity or visual phase separation and mixtures thereof.

2. The hair care composition according to claim 1, wherein the hair care composition further comprises
a. a gel matrix comprising:
i. from 0.1% to 20% of one or more high melting point fatty compounds, by weight of said hair care composition;
ii. from 0.1% to 10% a cationic surfactant system, by weight of said hair care composition; and
at least 20% of an aqueous carrier, by weight of said hair care composition.

3. The hair care composition according to any preceding claims, wherein the pre-emulsified emulsion comprises one or more water-miscible solvents.

4. The hair care composition according to any preceding claims, wherein the average particle size of the pre-emulsified emulsion is from 100 nm to 20 microns.

5. The hair care composition according to any preceding claims, wherein the average particle size of the metathesized unsaturated polyol esters in the hair care composition is from 100 nm to 20 microns.

6. The hair care composition according to any preceding claims, wherein said soy oligomer is fully hydrogenated.

7. The hair care composition according to any preceding claims, wherein said hair care composition further comprises one or more additional conditioning agents, preferably wherein the conditioning agent is a silicone.

8. The hair care composition according to any preceding claims, wherein said hair care composition further comprises a deposition polymer.

9. The hair care composition according to any preceding claims, wherein said hair care compositions comprises from 0.03% to 8% by weight of said hair care composition of a deposition polymer which is a copolymer comprising: a vinyl monomer (A) with a carboxyl group in the structure; and a vinyl monomer (B) expressed by the following formula (1):
CH₂=C(R¹)-CO-X-(Q-O)ᵣ-R² (1)
wherein: R¹ represents a hydrogen atom or a methyl group; R² represents a hydrogen atom or an alkyl group with from 1 to 5 carbon atoms, which may have a substitution group; Q represents an alkylene group with from 2 to 4 carbon atoms which may also have a substitution group; r represents an integer from 2 to 15; and X represents an oxygen atom or an NH group; and, in the following structure - (Q - O)ᵣ - R², the number of atoms bonded in a straight chain is 70 or less; and wherein the vinyl monomer (A) is contained at a level of from 10 mass% to 50 mass%, and the vinyl monomer (B) is contained at level of from 50 mass% to 90 mass%.

10. The hair care composition according to any of the claims 8 or 9, wherein said hair care composition comprises a weight ratio of (i) the deposition polymer to (ii) a sum of the mono-alkyl amine salt cationic surfactant, di-alkyl quaternized ammonium salt cationic surfactant, and high melting point fatty compound is from 1:1 to 1:160.

11. The hair care composition according to any preceding claims, wherein said metathesized unsaturated polyol esters are oligomers, wherein said one or more oligomers is self-metathesized, cross-metathesized or mixtures thereof.

12. The hair care composition according to any preceding claims, wherein said hair care composition further comprises one or more non-metathesized unsaturated polyol esters, preferably wherein the one or more non-metathesized unsaturated polyol esters is a soybean oil, a natural oil and mixtures thereof.

## Patentansprüche

1. Haarpflegezusammensetzung, umfassend:
a. zu 0,25 % bis 80 % eine voremulgierte Emulsion, umfassend zu 0,005 % bis 80 % ein oder mehrere Materialien, die aus der Gruppe ausgewählt sind, bestehend aus metathesierten ungesättigten Polyolestern mit einem Molekulargewicht größer als oder gleich 1.500, bezogen auf das Gewicht der Haarpflegezusammensetzung;
wobei die metathesierten ungesättigten Polyolester Oligomere sind, wobei das eine oder die mehreren Oligomere ein Triglyceridoligomer ist, wobei das Triglyceridoligomer ein Sojaoligomer ist; wobei ein Emulgator ausgewählt ist aus der Gruppe bestehend aus anionischem Emulgator, nichtionischem Emulgator, kationischem Emulgator, amphoterem Emulgator und Mischungen davon; wobei die durchschnittliche Teilchengröße der voremulgierten Öl-in-Wasser-Emulsion von 20 Nanometer bis 20 Mikrometer beträgt; und
b. ein kationenaktives Tensidsystem;
ferner wobei die Zusammensetzung in Bezug auf eines der folgenden Maße, ausgewählt aus Emulsionteilchengröße, Viskosität oder visuelle Phasentrennung und Mischungen davon, stabil ist.

2. Haarpflegezusammensetzung nach Anspruch 1, wobei die Haarpflegezusammensetzung ferner umfasst
a. eine Gelmatrix, umfassend:
i. zu 0,1 % bis 20 % ein oder mehrere Fettverbindungen mit hohem Schmelzpunkt, bezogen auf das Gewicht der Haarpflegezusammensetzung;
ii. zu 0,1 % bis 10 % ein kationenaktives Tensidsystem; bezogen auf das Gewicht der Haarpflegezusammensetzung; und
zu mindestens 20 % einen wässrigen Träger, bezogen auf das Gewicht der Haarpflegezusammensetzung.

3. Haarpflegezusammensetzung nach einem der vorstehenden Ansprüche, wobei die voremulgierte Emulsion ein oder mehrere wassermischbare Lösungsmittel umfasst.

4. Haarpflegezusammensetzung nach einem der vorstehenden Ansprüche, wobei die durchschnittliche Teilchengröße der voremulgierten Emulsion von 100 nm bis 20 Mikrometer beträgt.

5. Haarpflegezusammensetzung nach einem der vorstehenden Ansprüche, wobei die durchschnittliche Teilchengröße der metathesierten ungesättigten Polyolester in der Haarpflegezusammensetzung von 100 nm bis 20 Mikrometer beträgt.

6. Haarpflegezusammensetzung nach einem der vorstehenden Ansprüche, wobei das Sojaoligomer vollständig hydriert ist.

7. Haarpflegezusammensetzung nach einem der vorstehenden Ansprüche, wobei die Haarpflegezusammensetzung ferner ein oder mehrere zusätzliche Konditioniermittel umfasst, vorzugsweise wobei das Konditioniermittel ein Silikon ist.

8. Haarpflegezusammensetzung nach einem der vorstehenden Ansprüche, wobei die Haarpflegezusammensetzung ferner ein Abscheidepolymer umfasst.

9. Haarpflegezusammensetzung nach einem der vorstehenden Ansprüche, wobei die Haarpflegezusammensetzungen zu 0,03 % bis 8 %, bezogen auf das Gewicht der Haarpflegezusammensetzung, ein Abscheidepolymer umfasst, das ein Copolymer ist, umfassend: ein Vinylmonomer (A) mit einer Carboxylgruppe in der Struktur; und ein Vinylmonomer (B), ausgedrückt durch die folgende Formel (1):
CH₂=C(R¹)-CO-X-(Q-O)ᵣ-R² (1)
wobei: R¹ für ein Wasserstoffatom oder eine Methylgruppe steht; R² für ein Wasserstoffatom oder eine Alkylgruppe mit 1 bis 5 Kohlenstoffatomen steht, die eine Substitutionsgruppe aufweisen können; Q für eine Alkylengruppe mit 2 bis 4 Kohlenstoffatomen steht, die ebenfalls eine Substitutionsgruppe aufweisen können; r für eine ganze Zahl von 2 bis 15 steht; und X für ein Sauerstoffatom oder eine NH-Gruppe steht; und, in der folgenden Struktur - (Q - O)ᵣ - R², die Anzahl von in einer geraden Kette gebundenen Atomen 70 oder weniger beträgt; und wobei das Vinylmonomer (A) in einem Anteil von 10 Massen-% bis 50 Massen-% enthalten ist, und das Vinylmonomer (B) in einem Anteil von 50 Massen-% bis 90 Massen-% enthalten ist.

10. Haarpflegezusammensetzung nach einem der Ansprüche 8 oder 9, wobei die Haarpflegezusammensetzungen ein Gewichtsverhältnis (i) des Abscheidepolymers zu (ii) einer Summe des kationenaktiven Monoalkylaminsalz-Tensids, des kationenaktiven quaternisierten Dialkylammoniumsalz-Tensids und der Fettverbindung mit hohem Schmelzpunkt von 1 : 1 bis 1 : 160 umfasst.

11. Haarpflegezusammensetzung nach einem der vorstehenden Ansprüche, wobei die metathesierten ungesättigten Polyolester Oligomere sind, wobei das eine oder die mehreren Oligomere selbstmetathesiert, kreuzmetathesiert oder Mischungen davon ist.

12. Haarpflegezusammensetzung nach einem der vorstehenden Ansprüche, wobei die Haarpflegezusammensetzung ferner einen oder mehrere nicht metathesierte ungesättigte Polyolester umfasst, vorzugsweise wobei das eine oder die mehreren nicht metathesierten ungesättigten Polyolester ein Sojaöl, ein natürliches Öl und Mischungen davon ist.

## Revendications

1. Composition pour soins capillaires, comprenant :
a. de 0,25 % à 80 % d'une émulsion pré-émulsifiée comprenant de 0,005 % à 80 % d'une ou de plusieurs matières choisies dans le groupe constitué d'esters de polyol insaturés ayant subi une métathèse, avec un poids moléculaire supérieur ou égal à 1 500, en poids de ladite composition pour soins capillaires ;
dans laquelle lesdits esters de polyols insaturés obtenus par métathèse sont des oligomères, dans laquelle lesdits un ou plusieurs oligomères est un oligomère de triglycéride, dans laquelle l'oligomère de triglycéride est un oligomère de soja ; dans laquelle un émulsifiant est choisi dans le groupe constitué d'un émulsifiant anionique, d'un émulsifiant non ionique, d'un émulsifiant cationique, d'un émulsifiant amphotère et des mélanges de ceux-ci ; dans laquelle la taille moyenne des particules de l'émulsion d'huile dans l'eau pré-émulsifiée est de 20 nanomètres à 20 microns ; et
b. un système d'agent tensioactif cationique ;
dans laquelle en outre la composition est stable par rapport à l'une des mesures suivantes choisies parmi la taille des particules d'émulsion, la viscosité ou la séparation des phases visuelle et leurs mélanges.

2. Composition pour soins capillaires selon la revendication 1, dans laquelle la composition pour soins capillaires comprend en outre
a. une matrice de gel comprenant :
i. de 0,1 % à 20 % d'un ou plusieurs composés gras à haut point de fusion, en poids de ladite composition pour soins capillaires ;
ii. de 0,1 % à 10 % d'un système tensioactif cationique, en poids de ladite composition pour soins capillaires ; et
au moins 20 % d'un véhicule aqueux, en poids de ladite composition pour soins capillaires.

3. Composition pour soins capillaires selon l'une quelconque des revendications précédentes, dans laquelle l'émulsion pré-émulsifiée comprend un ou plusieurs solvants miscibles à l'eau.

4. Composition pour soins capillaires selon l'une quelconque des revendications précédentes dans laquelle la taille moyenne des particules de l'émulsion pré-émulsifiée est de 100 nm à 20 microns.

5. Composition pour soins capillaires selon l'une quelconque des revendications précédentes, dans laquelle la taille moyenne des particules des esters de polyol insaturés ayant subi une métathèse dans la composition pour soins capillaires est de 100 nm à 20 microns.

6. Composition pour soins capillaires selon l'une quelconque des revendications précédentes, dans laquelle ledit oligomère de soja est totalement hydrogéné.

7. Composition pour soins capillaires selon l'une quelconque des revendications précédentes, dans laquelle ladite composition pour soins capillaires comprend en outre un ou plusieurs agents de conditionnement supplémentaires, de préférence dans laquelle l'agent de conditionnement est un silicone.

8. Composition pour soins capillaires selon l'une quelconque des revendications précédentes, dans laquelle ladite composition pour soins capillaires comprend en outre un polymère de dépôt.

9. Composition pour soins capillaires selon l'une quelconque des revendications précédentes, dans laquelle ladite composition pour soins capillaires comprend de 0,03 % à 8 % en poids de ladite composition pour soins capillaires d'un polymère de dépôt qui est un copolymère comprenant : un monomère vinylique (A) avec un groupe carboxyle dans la structure ; et un monomère vinylique (B) exprimé par la formule (1) suivante :
CH₂=C(R¹)-CO-X-(Q-O)ᵣ-R² (1)
dans laquelle : R¹ représente un atome d'hydrogène ou un groupe méthyle ; R² représente un atome d'hydrogène ou un groupe alkyle avec de 1 à 5 atomes de carbone, qui peuvent avoir un groupe de substitution ; Q représente un groupe alkylène avec de 2 à 4 atomes de carbone qui peuvent également avoir un groupe de substitution ; r représente un nombre entier allant de 2 à 15 ; et X représente un atome d'oxygène ou un groupe NH ; et, dans la structure suivante - (Q - O)ᵣ - R², le nombre d'atomes liés dans une chaîne linéaire est 70 ou moins ; et dans laquelle le monomère vinylique (A) est contenu à un taux allant de 10 % en masse à 50 % en masse, et le monomère vinylique (B) est contenu à un taux allant de 50 % en masse à 90 % en masse.

10. Composition pour soins capillaires selon l'une quelconque des revendications 8 et 9, dans laquelle ladite composition pour soins capillaires comprend un rapport pondéral du (i) polymère de dépôt sur (ii) une somme de l'agent tensioactif cationique de sel monoalkylamine, d'un agent tensioactif cationique de sel dialkylammonium quaternarisé et d'un composé gras à haut point de fusion allant de 1:1 à 1:160.

11. Composition pour soins capillaires selon l'une quelconque des revendications précédentes, dans laquelle lesdits esters de polyol insaturés ayant subi une métathèse sont des oligomères, dans laquelle ledit ou lesdits oligomères sont des oligomères ayant subi une auto-métathèse, une métathèse croisée ou des mélanges de celles-ci.

12. Composition pour soins capillaires selon l'une quelconque des revendications précédentes, dans laquelle ladite composition pour soins capillaires comprend en outre un ou plusieurs esters de polyol insaturés n'ayant pas subi une métathèse, de préférence dans laquelle le ou les esters de polyol insaturés n'ayant pas subi de métathèse sont une huile de soja, une huile naturelle ou des mélanges de celles-ci.
